# EUROPEAN PATENT SPECIFICATION

(11) **EP 1 458 247 B1**
(45) Date of publication and mention of the grant of the patent: **22.01.2014**
(21) Application number: 02787279.5
(22) Date of filing: 20.12.2002
(51) Int. Cl.: A23J 3/08, A23J 3/22, A23L 1/30, A23L 1/24, A23C 21/02, A23C 15/16, A23C 9/137, A61K 47/42

(54) **MALLEABLE PROTEIN MATRIX AND USES THEREOF**
VERFORMBARE PROTEINMATRIX UND ANWENDUNGEN
MATRICE PROTEIQUE MALLEABLE ET SES UTILISATIONS

(30) Priority: 20.12.2001 US 341232 P
(43) Date of publication of application: 22.09.2004
(73) Proprietor: Biolactis Inc., Nassau (BS)
(72) Inventor: SIMARD, Eric, Laval, Québec H7L 5Z7 (CA); PILOTE, Dominique, Chicoutimi, Québec G7G 1M5 (CA); DUPONT, Claude, Blainville, Québec J7B 1L6 (CA); LAJOIE, Nathalie, Jonquière, Québec G7S 5Z7 (CA); PAQUET, Marcel, Chicoutimi, Québec G7H 2Y6 (CA); LEMIEUX, Pierre, Ste-Thérèse, Québec J7E 4Z2 (CA); GOYETTE, Philippe, Montréal, Québec H2L 2T1 (CA)
(74) Representative: Jacob, Reuben Ellis
(86) International application number: PCT/CA2002/001988
(87) International publication number: WO 2003/053158

(56) References cited:
- EP-A- 0 498 506
- WO-A-92/06598
- US-A- 4 183 966
- US-A- 4 444 793
- US-A- 4 699 793
- US-A- 5 756 136
- CHRISTIANSEN P S ET AL: "SLIME FORMATION IN WHEY BY ROPY LACTOCOCCUS LACTIS SSP. CREMORIS322 SCHLEIMBILDUNG DURCH FADENZIEHENDEN LACTOCOCCUS LACTIS SSP. CREMORIS322 IN MOLKE" MILCHWISSENSCHAFT, VV GMBH VOLKSWIRTSCHAFTLICHER VERLAG. MUNCHEN, DE, vol. 56, no. 10, 2001, pages 545-548, XP001114938 ISSN: 0026-3788
- GOMES A M P ET AL: "Bifidobacterium spp. and Lactobacillus acidophilus: biological, biochemical, technological and therapeutical properties relevant for use as probiotics." TRENDS IN FOOD SCIENCE & TECHNOLOGY 10 (4/5) 139-157 1999 CORRESPONDENCE (REPRINT) ADDRESS, F. XAVIER-MALCATA, ESCOLA SUPERIOR DE BIOTEC., UNIV. CATOLICA PORTUGUESA RUA, DR. ANTONIO BERNARDINO DE ALMEIDA, 4200 PORTO, PORTUGAL. FAX +351-2-5090351, XP002242210 cited in the application
- PERDIGON ET AL.: "Lactic acid bacteria and their effect on the immune system" CURRENT ISSUES IN INTESTINAL MICROBIOLOGY, 2001, page 27-42 XP008017417 GB cited in the application

## Description

### BACKGROUND OF THE INVENTION

### (a) Field of the invention

This invention relates to a biodegradable and natural malleable protein matrix, method of preparation thereof, and compositions thereof, such as food, cosmetic, nutraceutical, functional food and pharmaceutical compositions.

### (b) Description of Prior Art

The high demand for low fat product lead the food industry to develop substitute food. The high demand for such product is based on studies recommending a decreased in daily fat consumption, It is important for the substitute food to have interesting sensorial characteristics as the original food (taste, smell, texture, etc.). Another field being in intensive increase Is the nutraceutical and functional foods. The functional food is food with beneficial effect on health. The world consumption of these new foods is of about 70MM$ on an annual basis. The popularity of these products is so high that worldwide sales are expected to be of 500MM$ in 2010.

In the cosmetic and pharmaceutical industry, there is a long felt need for raw material for formulations, protection and controlled liberation of active ingredients. Several products are already existing, but most of them are very expensive. The industry is always in need for new technologies and products that will produce better results at a lower cost.

Ultrafiltration, reverse osmosis and drying processes are among the methods currently used for the valorization of whey proteins from the so-called serum lactis, the by-product of cheese making. These methods are efficient but extremely expensive and do not generate a readily useable product In a variety of industrial sectors. The fact that the cost of the installations for the above-mentioned methods is high is a problem for the cheese industry in general. Only large cheese manufacturers with strong financial positions and generating large volumes of serum lactis can reach profitability with the above-mentioned methods despite the high costs. Since serum lactis cannot be discarded freely in the environment it constitutes a pollutant *per se,* the small manufacturers have therefore to spend money to discard the serum lactis which is mainly used for animal feed.

Simpler and less costly processes were developed to retrieved whey proteins but with also with concomitant drawbacks. Methods using temperature, pH, salt, enzymes, fermentation and flocculent are among the main parameters used to help the retrieval of whey proteins but generally lead to isolates exhibiting poor commercial quality and value. Patent CA 2,247,824 by Lewandoski and co-inventors describes a process for the production of microbial biomass from the effluent of dairy products. The resulting biomass from that process is used for animal feeding only. However, this product is not having functional properties such as emulsifiant properties that are needed for applications in human food.

Many processes and methods are offered to replace fat in food products. Agglomerates of whey proteins are used to replace fat like as described in U.S. Patent 5,358,730. The process involves a thermal treatment of whey proteins at a pH above their isoelectric point with the addition of salt. The process leads to the formation of curds (solid gels that can be shopped off in little pieces) that can be used in fat replacement. Whey proteins are extensively used in the food industry for their functional properties. However, this product is a solide and non-malleable product..that is difficult to use in most of the food, cosmetic, pharmaceutical and nutraceuticals applications.

Proteins are also excellent film formers, conditioning agents, and moisturizers for hair and skin. However, natural proteins generally have limited use in cosmetics and toiletries because they are somewhat unstable and tend to precipitate or denature when exposed to high temperatures or salt solutions. In addition they are often hydrolyzed by chemical reagents or acids and bases. Even if these difficulties are overcome, the formulation of cosmetic' products containing proteins is further fraught with difficulty since each protein has an isoelectric point i.e. a pH at which the protein is neutral. If it is desired to form compositions having a pH which is below the isoelectric point of the protein, the protein may possibly form an insoluble precipitate.

Furthermore, a large number of food products like mayonnaise, dressings, margarine, spreads or low-fat or zero-fat substitutes, can be stabilized by polysaccharides as emulsion stabilizers or thickening agents. Also in the the medical, pharmaceutical and cosmetic fields, polysaccharides they are used as emulsion stabilizers. Well known polysaccharides are obtained from a variety of plant seeds, e.g. guar gum from Cyamopsis tetragonaloba (guar) or locust bean gum (LBG) from locust bean. Other well-known sources are seaweed, giving carrageenan, alginates or agar.

The use of polysaccharides and proteins in cosmetic compositions is well known in the art. Polysaccharides are known to be good humectants, film formers, and function as skin moisturizers. Certain polysaccharides also have gelling ability and are useful in formation of higher viscosity liquid or solid, compositions. However, polysaccharides may tend to provide a heavy, sticky feel on the skin and, when used in quantities sufficient to cause gelling, may provide products which are not aesthetically pleasing.

### Food science

The process described in the U.S. Patent 4,699,793 is used to produce seasoning. Because of the heat treatment performed before the fermentation, the resulting product has an undesirable taste and a poor homogeneity, which are the most important parameters in food science.

It is known that the presence of certain bacteria is associated with numerous beneficial effects on health (Gomes et al. (1999) T. Food Sc. & Tech. 10:139-157). The microorganisms are present in many foods and are frequently used as probiotics to improve some biological functions in the host. Clinical trials have demonstrated that selected probiotic strains can influence the composition of the intestinal microflora and modulate the host immune system. Pre-, pro- and synbiotics offer both protection against and cure a variety of endemic and acute diseases.

More particularly, the lactic acid bacteria (LAB) are known for their several beneficial effects on health. Perdigon et al. (Curr Issues Intest Microbiol., 2001, Mar 2(1):27-42), have proceed with an important review of the lactic bacteria on health, particularly on immune system. The activation of the systemic and secretory immune response by LAB requires many complex interactions among the different constituents of the intestinal ecosystem (microflora, epithelial cells and immune cells). Through different mechanisms they send signals to activate immune cells. Thus the knowledge of the normal intestinal microflora, the contribution of LAB and their role in the numerous functions in the digestive tract as well as the functioning of the mucosal immune system form the basis for the study and selection of a probiotic strain with immunostimulatory properties. In the selection of LAB for their immunostimulatory capacity it helps to know not only the effect which they have on the mucosal immune system, but the specific use to which these oral vaccine vectors are being put.

EP 0 498 506 describes a mixed lactic fermenting agent comprising at least two yeasts and at least two bacteria in a polysaccharide matrix. A lactoserum is fermented in the presence of the fermenting agent in order to obtain a lactic leaven that is used in the preparation of food products.

US 4,444,793 relates to a method for producing a functionalised dairy whey product containing a thickening polymer for use in the food industry. A fermentation broth of whey and sucrose is formed and fermented with the organism *Leuconostoc mesenteroides* ATCC 14935.

US 4,699,793 relates to a method for producing a seasoning from whey. A first step is described in which multiplication factors (i.e. vitamins, yeast extract, skim milk and lactulose) and L-cysteine hydrochloride are added to whey and the mixture fermented.

### Pharmaceutical

Delivery of therapeutic agents to a mammalian host can frequently be as important as the activity of the drug in providing effective treatment. For the most part, drugs are delivered orally, frequently initially at a dosage below the therapeutic dosage and by repetitive administration of the drug, the dosage is raised to a therapeutic level or a level exceeding the therapeutic level. In many cases, the fact of having a dosage above therapeutic level provides for adverse effects, since most drugs are not only effective for the intended purpose, but frequently have adverse side effects. Various proposals have been made to avoid these problems, such as slow-release capsules, depots, pumps, and the like. These various approaches have numerous short comings for general applications where one wishes to maintain the presence of a therapeutic agent at a therapeutic dosage 'for an extended period. Invasive procedures are frequently undesirable, requiring surgery for introduction of the delivery device, followed by subsequent removal. Where the delivery device is placed on the skin, the agent must be capable of transport across the skin at the desired rate. Slow release particles have a limited time span and when introduced into the blood stream will be rapidly phagocytosed.

Oral administration in the form of a conventional tablet, pill or capsule constitutes the generally preferred route for administration of pharmaceuticals since this route is generally convenient and acceptable to patents. Unfortunately such compositions may be associated with certain disadvantages, particularly in the treatment of pediatric or geriatric patients, who may dislike or have difficulty in swallowing such compositions, or where administration of a conventional tablet, pill or capsule is not duable.

The field of biodegradable polymers has developed rapidly since the synthesis and biodegradability of polylactic acid was first reported by Kulkami et al., 1966 "Polylactic acid for surgical implants" Arch. Surg., 93:839, Several other polymers are known to biodegrade, including polyanhydriques and polyorthoesters, which take advantage of labile backbone linkages, as reported by Heller et al., 1990, Biodegradable Polymers as Drug Delivery Systems; Chasin, M. and Langer, R., Eds., Dekker, New York, 121-161. Since it is desirable to have polymers that degrade into naturally occurring materials, polyaminoacids have been synthesized for *in vivo* use. This was the basis for using polyesters of alpha-hydroxy acids (viz, lactic acid, glycolic acid), which remain the most widely used biodegradable materials for applications ranging from closure devices (sutures and staples) to drug delivery systems (U.S. Pat. No. 4,741,337 to Smith et al.; Spilizewski et al., 1985 "The effect of hydrocortisone loaded poly(dl-lactide) films on the inflammatory response, " J. Control. Rcl. 2:197-203). Despite the development of novel biodegradable polymers, there is still a need for inexpensive and efficient delivery systems.

Exopolysaccharides act as biological response modifier as reported by Ruiz-Bravo A. (Clinical and Diagnostic Laboratory Immunology 2001, Jul; 8(4)-706-10). U.S. patents 5,888,552; 5,456,924; 5,451,412; 5,290,571; 5,230,902 describe compositions and methods to improve immune responses at large either for cancer or HIV-patients. U.S. patent 5,888,552 describes anti-cancer therapeutic compositions containing whey proteins while U.S. 5,456,924 describes a method of treatment of HIV-seropositive individual with dietary whey proteins.

It would be highly desirable to be provided with a biodegradable and non-toxic malleable protein matrix and a process to produce such that would turn or convert an industrial waste into a product with a commercial value and a biological activity.

### SUMMARY OF THE INVENTION

One object of the present invention is to provide a biodegradable and non-toxic malleable protein matrix (MPM).

Preferably, the invention relates to matrix of whey proteins and expopolysaccharides. In addition, the matrix of the present invention is advantageously used to replace fat or for the incorporation or encapsulation of various hydrophilic or lipophylic substances and particularly substances used in the food, cosmetic, nutraceuticals and pharmaceutical sectors.

Another object of the present invention, there is to provide a novel method for the retrieval of whey proteins from the serum lactis which leads to a new kind of whey protein-based product. This new product is referred hereto as a malleable protein matrix (MPM), which is the reaction product of the agglomeration of whey proteins present in the serum lactis after a fermentation process. It has the texture of a malleable cream exhibiting biological activities and unique properties for the incorporation (or encapsulation) of various hydrophilic or lipophylic substances.

It is also an object of the invention to prepare various types of MPMs with different properties, characteristics and multiple applications and to prepare them directly from an industrial waste (whey or serum lactis).

In accordance with the present invention, there is provided a process for manufacturing a malleable protein matrix, said process comprising the steps of:
a) fermenting a whey protein solution with bacteries in a medium;
b) agglomerating proteins from the protein solution of step a); and
c) isolating agglomerated proteins from supernatant;
characterised in that said bacteries comprises at least one bacteria selected from R2C2, INIX, ES 1 and K2.

The process in accordance with a preferred embodiment of the present invention, wherein said fermenting step is promoted by co-culturing at least two microorganisms simultaneously or successively.

The process in accordance with a preferred embodiment of the present invention, wherein said process further comprises a step between steps a) and b) for addition of a polysaccharide.

The process in accordance with a preferred embodiment of the present invention, wherein said process further comprises a step between steps b) and c) for addition of a polysaccharide.

The process in accordance with a preferred embodiment of the present invention, wherein agglomeration of fermented proteins is effected by at least one method selected from the group consisting of salt addition, pH modulation, thermal treatment, proteolytic enzymes addition and flocculent addition.

The process in accordance with a preferred embodiment of the present invention, further comprising a step of pasteurization of said proteins solution before step a).

The process in accordance with a preferred embodiment of the present invention, wherein said step of pasteurization is followed by a step of sterilization.

The process in accordance with a preferred embodiment of the present invention, wherein said flocculent is a bacterial flocculent.

The process in accordance with a preferred embodiment of the present invention, wherein said bacterial flocculent is *L. Kefirgranum.*

The process in accordance with a preferred embodiment of the present invention, wherein separation of agglomerated proteins from supernatant is effected by a method selected from the group of centrifugation and filtration.

In accordance with the present invention, there is provided a malleable protein matrix obtainable by the process of the present invention.

The matrix in accordance with a preferred embodiment of the present invention, further comprising a fermentation by-product of the fermentation of said solution containing said protein by said bacteria.

The matrix in accordance with a preferred embodiment of the present invention, further comprising a peptide.

The matrix in accordance with a preferred embodiment of the present invention, wherein said peptide comprises at least two amino acid residues.

The matrix in accordance with a preferred embodiment of the present invention, wherein said peptide comprises more than one hundred amino acid residues.

The matrix in accordance with a preferred embodiment of the present invention, wherein said fermentation by-product is a polysaccharide.

The matrix in accordance with a preferred embodiment of the present invention, wherein said polysaccharide is selected from the group of exopolysaccharide and anionic polysaccharide.

The matrix in accordance with a preferred embodiment of the present invention, wherein said polysaccharide contains at least four saccharide moieties.

The matrix in accordance with a preferred embodiment of the present invention, wherein said saccharide moieties are selected from the group consisting of D and L forms of glucose, fructose, xylose, arabinose, fucose, galactose, pyruvic acid, succinic acid, acetic acid, 3,6-anhydrogalactose sulfate, galactose-4-sulfate, galactose-2-sulfate, galactose-2, 6-disulfate, mannose, glucuronic acid, mannuronic acid, guluronic acid, galactouronic acid, and rhainnose.

The matrix in accordance with a preferred embodiment of the present invention, wherein said polysaccharide have molecular weight ranging from about 500 to about 15,000,000 daltons.

The matrix in accordance with a preferred embodiment of the present invention, wherein said molecular weight is ranging from about 5,000 to 6,000,000 daltons.

The matrix in accordance with a preferred embodiment of the present invention, wherein said molecular weight is ranging from about 25,000 to 1,000,000 daltons.

The matrix in accordance with a preferred embodiment of the present invention, wherein said polysaccharide is selected from the group consisting of heteropolysaccharide, homopolysaccharide and mixture thereof.

The matrix in accordance with a preferred embodiment of the present invention, wherein said heteropolysaccharide is selected from the group consisting of gellan, welan, gum arabic, karaya gum, okra gum, aloe gum, gum tragacanth, gum ghatti quicessed gum, psyllium, galactans, galactomannans, glucomannans, polyuronic acids, dextran sulfate, heparin, pectin, sodium alginate and starch arabinogalactan.

The matrix in accordance with a preferred embodiment of the present invention, wherein said galactan is selected from the group consisting of agar, agarose, kappa, carageenan, iota carageenan and lambda carageenan.

The matrix in accordance with a preferred embodiment of the present invention, wherein said galactomannan is selected from the group consisting of locust bean gum and guar.

The matrix in accordance with a preferred embodiment of the present invention, wherein said glucan is selected from the group consisting of cellulose and derivatives thereof, starch and derivatives, dextrans, pullulan, beta 1,3-glucans, chitin, xanthan and tamatind.

The matrix in accordance with a preferred embodiment of the present invention, wherein said glycomannan is konjac.

The matrix in accordance with a preferred embodiment of the present invention, wherein said polyuronic acid is selected from the group consisting of algin, alginate and pectin.

The matrix in accordance with a preferred embodiment of the present invention, wherein said homopolysaccharide is cellulose.

The matrix in accordance with a preferred embodiment of the present invention, further comprising one or more bacteria is selected from the group consisting of *Bifidobacterium adolescentis, Bifidobacterium angulatum, Bifidobacterium animalis, Bifidobacterium asteroides, Bifidobacterium bifidum, Bifidobacterium boum, Bifidobacterium breve, Bifidobacterium catanulatum, Bifidobacterium choerinum, Bifidobacterium coryneforme, Bifidobacterium cuniculi, Bifidobacterium dentium, Birdobacterium gallicum, Bifidobacterium gallinarum, Bifidobacterium indicum, Bifidobacterium infantis, Bifidobacterium longum, Bifidobacterium longum* DJO1OA, *Bifidobacterium longum* NCC2705, *Bifidobacterium magnum, Bifidobacterium merycicum, Bifidobacterium minimum, Bifidobacterium pseudocatenulatum, Bifidobacterium pseudolongum, Bifidobacterium pseudolongum subsp. globosum, Bifidobacterium pullorum, Bifidobacterium ruminantium, Bifidobacterium saeculare, Bifidobacterium scardovii, Bifidobacterium subtile, Bifidobacterium suis, Bifidobacterium thermacidophilum, Bifidobacterium thermacidophilum subsp. suis, Bifidobacterium thermophilum, Bifidobacteilum urinalis, Lactobacillus acetotolerans, Lactobacillus acidipiscis, Lactobacillus acidophilus, Lactobacillus agilis, Lactobacillus algidus, Lactobacillus alimentarius, Lactobacillus amylolyticus, Lactobacillus amylophilus, Lactobacillus amylovorus, Lactobacillus animalis, Lactobacillus arizonenis, Lactobacillus aviarius, Lactobacillus bifermentans, Lactobacillus brevis, Lactobacillus buchneri, Lactobacillus casei, Lactobacillus cellobiosus, Lactobacillus coleohominis, Lactobacillus collinoides, Lactobacillus coryniformis, Lactobacillus coryniformis subsp. coryniformis, Lactobacillus corniformis subsp. torquens, Lactobacillus crispatus, Lactobacillus curvatus, Lactobacillus cypricasei, Lactobacillus delbrueckii, Lactobacillus delbrueckii subsp. bulgaricus, Lactobacillus delbrueckii subps. delbrueckii, Lactobacillus delbrueckii subsp. lactis, Lactobacillus durianis, Lactobacillus equi, Lactobacillus farciminis, Lactobacillus ferintoshensis, Lactobacillus fermentum, Lactobacillus fornicalis, Lactobacillus fructivorans, Lactobacillus frumenti, Lactobacillus fuchuensis, Lactobacfllus gallinarum, Lactobacillus gasseri, Lactobacillus graminis, Lactobacillus hamsteri, Lactobacillus helveticus, Lactobacillus helveticus subsp. jugurti, Lactobacillus heterohiochii, Lactobacillus hilgardii, Lactobacillus homohiochii, Lactobacillus intestinalis, Lactobacillus japonicus, Lactobacillus jensenii, Lactobacillus johnsonii, Lactobacillus kefer, Lactobacillus kefiri, Lactobacillus kefiranofaciens, Lactobacillus kefirgranum, Lactobacillus kimchii, Lactobacillus kunkeei, Lactobacillus leichmannii, Lactobacillus letivazi, Lactobacillus lindneri, Lactobacillus malefermentans, Lactobacillus mali, Lactobacillus maltaromicus, Lactobacillus manihotivorans, Lactobacillus mindensis, Lactobacillus mucosae, Lactobacillus murinus, Lactobacillus nagelii, Lactobacillus oris, Lactobacillus panis, Lactobacillus pantheris, Lactobacillus parabuchneri, Lactobacillus paracasei, Laetobacillus paracasei subsp. paracasei, Lactobacilhus paracasei subsp. tolerans, Lactobacillus parakefri, Lactobacillus paralimentarius, Lactobacillus paraplantarum, Lactobacillus pentosus, Lactobacillus perolens, Lactobacillus plantarum, Lactobacillus pontis, Lactobacillus psittaci, Lactobacillus reuteri, Lactobacillus rhamnosus, Lactobacillus ruminis, Lactobacillus sakei, Lactobacillus sakei* L45, *Lactobacillus salivarius, Lactobacillus salivarius subsp. salicinius, Lactobacillus salivarius subsp. salivarius, Lactobacillus sanfranciscensis, Lactobacillus sharpeae, Lactobacillus sp.* NGRI 0001, *Lactobacillus suebicus, Lactobacillus thermotolerans, Lactobacillus vaccinostercus, Lactobacillus vaginalis, Lactobacillus vermtiforme, Lactobacillus versmoldensis, Lactobacillus zeae, Lactococcus gameae, Lactococcus lactis, Lactococcus lactis subsp. cremoris, Lactococcus lactis subsp. hordniae, Lactococcus lactis subsp. lactis, Lactococcus latis subsp. lactis bv. diacetylactis, Lactococcus piscium, Lactococcus plantarum, Lactococcus raffinolactis, Leuconostoc argentinum, Leuconostoc camosum, Leuconostoc citreum, Leuconostoc fallax, Leuconostoc ficulneum, Leuconostoc fructosum, Leuconostoc gasicomitatum, Leuconostoc gelidum, Leuconostoc inhae, Leuconostoc kimchii, Leuconostoc lactis, Leuconostoc mesenteroides, Leuconstoc niesenteroides subsp. cremoris, Leuconostoc mesenteroides subsp. dextranicum, Leuconostoc mesenteroides subsp. mesenteroides, Leuconostoc mesenteroides subsp. mesenteroides* ATCC 8293, *Leuconostoc pseudomesenteroides, Propionibacterium acidipropionici, Propionibacterium acnes, Propionibacterium australiense, Propionobacterium avidum, Propionibacterium cyclohexanicum, Propionibacterium freudenreichii, Propionibacterium freudenreichii subsp. freudenreichii, Propiontbacterium freudenreichii subsp. shermanii, Propionibacterium granulosum, Propionibacterium jensenii, Propionobacterium lumpliophilum, Propionibacterium microaerophilum, Propionibacterium propionicum and Propionibacterium thoenii.*

In accordance with the present invention, there is provided a microorganism R2C2 isolated from a consortium obtained from Kefir grain under NML accession number 041202-3.

In accordance with the present invention, there is provided a microorganism K2 isolated from a consortium obtained from Kefir grain under NML accession number 041202-1.

In accordance with the present invention, there is provided a microorganism ES1 isolated from a consortium obtained from Kefir grain under NML accession number 041202-2.

In accordance with the present invention, there is provided a microorganism INIX isolated from ATCC 43761 strain.

In accordance with the present invention, there is provided a composition comprising the matrix of the present invention in association with a pharmaceutically acceptable carrier.

In accordance with the present invention, there is provided the use of the matrix of the present invention for the manufacture of a product selected from the group of food product, medical product, pharmaceutical product, cosmetic product and nutraceutical.

In accordance with the present invention, there is provided the use of the matrix of the present invention for the manufacture of a food product. Preferably, said matrix is used as an emulsion stabilizer or thickening agent. Preferably, said food product is selected from the group consisting of mayonnaise, dressing, margarine, spread, butter, whipped cream and low-fat substitute. Preferably, said matrix is used as a delivery vehicle.

In accordance with the present invention, there is provided the use of the matrix of the present invention, wherein said use is for cosmetic product. Preferably, said cosmetic product is selected from the group consisting of skin lotion, cream, sunscreen, blush, mascara, eyeshadow, shampoo and conditioner.

In accordance with the present invention, there is provided the use of the matrix of the present invention for the manufacture of a medicament for increasing immune response in a subject.

In accordance with the present invention, there is provided the use of the matrix of the present invention for the manufacture of a medicament for reducing triclyceride level in a subject.

In accordance with the present invention, there is provided the use of the matrix of the present invention for the manufacture of a medicament for reducing TNF-α level in a subject.

In accordance with the present invention, there is provided the use of the matrix of the present invention for the manufacture of a medicament for increasing gluthatione level in a subject.

The MPM of the present invention also fulfill a long-felt need in different sectors, namely in food (fat replacement, thickening agent), cosmetic (delivery systems, physiological effects), nutraceuticals, functional food, probiotic and pharmaceutical (oral delivery systems, biological response modifier drug delivery systems).

### BRIEF DESCRIPTION OF THE DRAWINGS

Fig. 1 illustrates a general schema of the preparation process for MPM;
Fig. 2 illustrates a detailed schema of the matrix formation;
Fig. 3 illustrates the formulation of matrix formulation;
Fig. 4 illustrates one example of an industrial implementation of the present invention; and
Figs. 5A-B illustrate homology between gene ARN165 of strains INIX (SEQ ID NO : 1), K2 (SEQ ID NO : 2), R2C2 (SEQ ID NO : 3), ES1 (SEQ ID NO : 4), ATCC 43761 (SEQ ID NO : 5) and ATCC 51647 (SEQ ID NO : 6).

### DETAILED DESCRIPTION OF THE INVENTION

The invention consists in a malleable protein matrix (MPM) produced from fermented residual whey obtained from the cheese industry. The MPM is obtained by triggering agglomeration of whey proteins, which are then retrieved by various means. The process allows the production of insoluble and malleable protein matrix composed of 1) proteins and/or peptides, 2). one or several bacterial strains, 3) fermented by-products, 4) other components obtained during the agglomeration and retrieval process of the agglomerates and 5) components present in the aqueous phase. Following the agglomeration, the resulting matrix is retrieved by filtration, centrifugation or with any other methods allowing such retrieval. The protein agglomeration can be triggered by, but not limited to, a modulation of pH, temperature, the addition of salts, the addition of proteolytic enzymes, the addition of flocculent or the combination of all or some of those methods. The invention also describes various parameters that can affect the resulting characteristics of the matrix like the bacterial component of the MPM.

.This matrix. and its production process present major advantages over the matrix and production processes known in the art. The production process as described below allows the obtention of a uniform formulation directly from lactoserum or other primary protein source when all the components are present prior agglomeration. The components are found either in the agglomerate and the aqueous phase of the post-agglomeration fermentation product. A formulation containing MPM is produced in mixing the MPM and other products to have introduced in the formulation in water, oil or other liquid suitable for such formulation. Another formulation is produced in lyophilizing MPM and hydrating them with a solution containing other products to be introduced in the formulations.

The polymers used can be from different origins, such as from a microorganism, from plant and they also can be synthetic. The polymer is being mixed to the proteins before, during or after the process of agglomeration. The amount of polymers trapped in the matrix may vary to form the resulting matrix. The source of proteins used in the agglomeration process can be from either pure whey obtained from a cheese factory or from a concentrate of whey proteins (WPC, CPI) resuspended in an aqueous solution. The agglomeration process is preceded by a fermentation process to improve the quality of the final product obtained: flavor, color, texture, conservation time, functional properties, nutritional properties, biological properties, pharmaceutical properties.

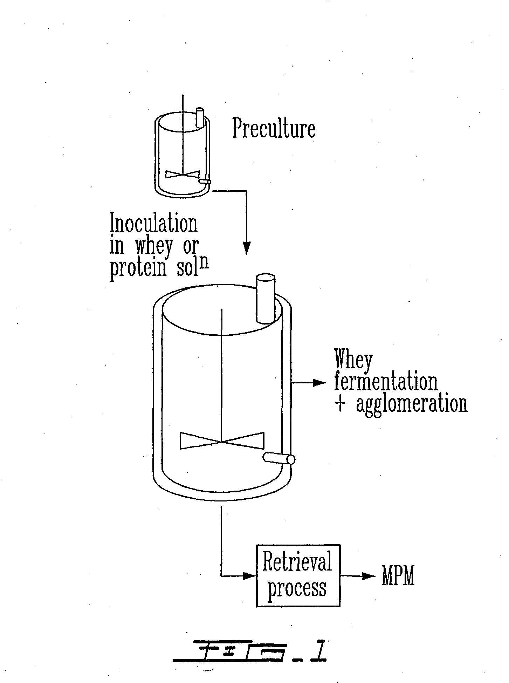

Fig. 1 illustrates the preferred embodiment of the process of the present invention consisting in a fermentation process of whey with a pure strain of lactobacillus isolated from a consortium obtained from Kefir grain (R2C2 strain accession number: 041202-3 National Microbiology Laboratory, Health Canada, 1015 Arlington Street, Winnipeg, Manitoba, Canada, R3E 3R2). The first step is a pre-culture where freeze-dried, or frozen ferment culture is used to inoculate whey or seed medium suitable for the species used like pure strain of lactobacillus isolated from a consortium obtained from Kefir grain (R2C2 strain). The fermentation is continued to get a concentration of bacteria of 10⁸ to 10⁹ bacteria per ml of pre-culture. The pre-culture is then inoculated in whey or protein solution in an amount of from 1 to 12,5%. Whey may be used as is, or supplemented with different culture additives suitable for the species used. During the proliferation process, the lactobacillus produces an exopolysaccharide (EPS), which is secreted in the medium, along with some endogenous proteases. The endogenous proteases present in the medium hydrolyze the whey proteins to generate peptides with various length and hydrophobicity. The whey medium is maintained under appropriate culture conditions to promote a rapid multiplication of the microorganisms used. If needed, a constant temperature, pH, agitation, aeration and other culture conditions are supplied. For triggering agglomeration of whey proteins, several means can be used to facilitate or inducing the formation of agglomerates like pH modification, salt addition and heat treatment. Agitation is needed to provide good homogeneity of the resulting matrix which contain microorganisms, peptides, proteins, fermented by-products. Continuous centrifugation is preferred to promote a better homogeneity of the matrix but various retrieval means can also be used.

MPM can be used under a humid form or dried and can be lyophilized or dried by other means and once dried the MPMs are also compressible with a Carver press to form solid tablets. Lyophilized MPMs are compressible without the need to add any excipients to form tablets that could have multiple applications like incorporation of probiotics or drugs. The tablets hydrate slowly because of their high content of proteins and are protecting the incorporated agents while passing in the stomach environment. MPM can integrate water, oil or other solvent to improve its general properties. The compositions and/or formulations obtained are useful in food science, cosmetic, nutraceuticals, functional food, probiotic and pharmaceuticals.

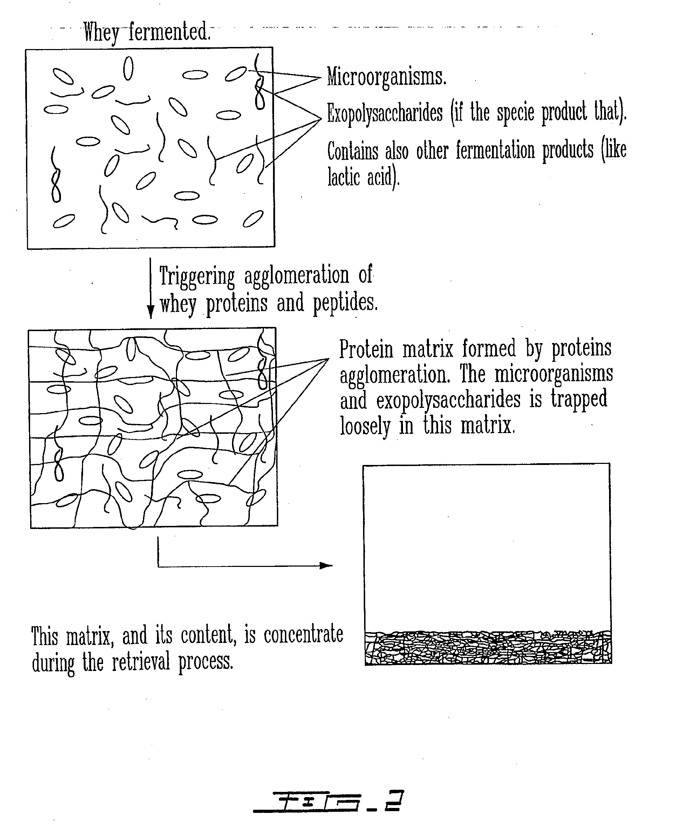

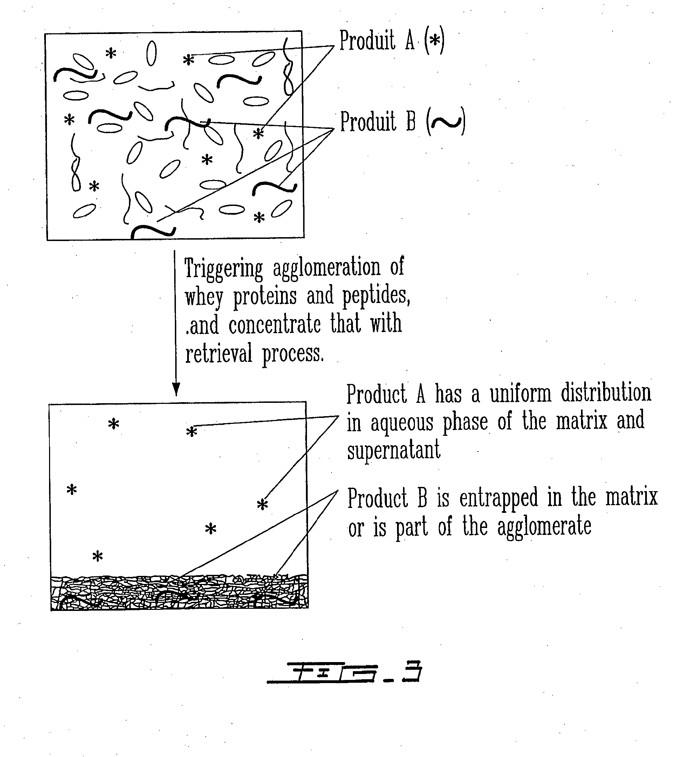

Fig. 2 illustrates the matrix formation and Fig. 3 illustrates formulations produced with MPM.

The process described to produce MPMs is preferably made of non-concentrated sources of whey proteins like serum lacis. The MPMs exhibit an improved homogeneity and a product with improved functional and organoleptic properties as well as beneficial effects on health because the fermentation process of the present invention is performed in a non- concentrated solution. There is therefore no need to homogenize the resulting matrix with high shear conditions as for the processes known in the art.

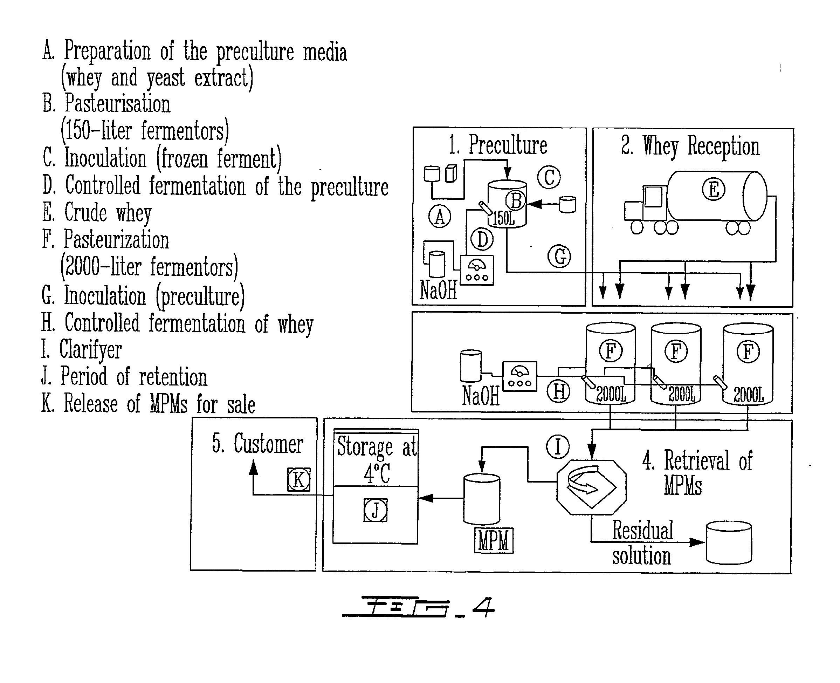

Fig. 4 illustrates an example of an industrial implementation of the process of the present invention. As shown in Fig. 4, a preculture medium is prepared with whey and yeast extract, followed by the pasteurization of this preparation. The pasteurized solution is then inoculated with ferment and fermented under control to the obtention of a bacterial culture of 108-109 bacteria per ml of preculture. One person skilled in the art would understand that the preculture medium preparation does not need to be part of the production process.

Whey is then provided in fermentor to which is added the preculture medium for fermentation. After completion of the fermentation process, the agglomeration of the fermented proteins is achieved by one or more of the methods previously described and the agglomerated proteins are isolated from the supernatant and stored until delivery.

The MPMs described above have multiple applications that are listed below. MPMs is an inexpensive product with a variety of competitive advantages and applications. In the food industry/functional food/nutraceuticals, the MPMs can be used as a fat replacement agent, as a protein supplement, as a functional food product having a specific feature (stimulation of the immune system, decreasing levels of triglyceride), as a bio-vehicle for ingredients, flavors, supplements, food additives, vitamins, In the cosmetic and as a cosmeceutical, the MPM can be used as fat and/or petroleum replacement agent, as a protein supplement in body lotion and cream, as a cosmeceutic product having specific features (increase *in situ* production of collagen), as a blo-vehicle for therapeutic agent, supplements, and vitamins. From the pharmaceuticals point of view, the MPMs can be used as a bio-vehicle for therapeutic agents, to increase oral formulation or generic drugs (excipient), to improve therapeutic indices of drugs (synergy), to reduce drug side effects and to increase bioavailability.

### Proteins

Although the preferred source of protein of the invention is the serum lactis, the process can also be applied to diluted protein solution. The term "protein" when used in accordance with this invention means a peptide chain having at least two amino acid residues, preferably at least four, and more preferably more than one hundred amino acid residues. Most preferably the protein is a high molecular weight polypeptide which is preferably water soluble.

The source of protein used in the invention is whey. Preferred is whey which is a mixture of whey proteins obtained from cow's milk following the cheese making.

MPM can become a solution to the difficulties encountered in the formulation of proteins. MPM can be formulated in creams for instance under either acidic or alkaline conditions without affecting the texture and appearance of the cream.

### Polymers

Several polymers may be used in the production of MPM. They are either synthetic or natural. However a variety of exopolysaccharides and polysaccharides are suitable for the preparation of the MPM used in the compositions of the invention, provided that the exopolysaccharides and polysaccharide contains a sufficient number of hydrophilic groups to cause the resulting MPM. In addition, the polysaccharide must be capable of reacting with the protein to form an MPM having a protein/polysaccharide ratio enough to cause aggregation. The term "polysaccharide" when used in accordance with the invention means a polysaccharide which contains at least four saccharide moieties. The term "saccharide moiety" means a polyhydroxy aldehyde or ketone, or acid hydrolysis product thereof, which, preferably, has the general formula Cₓ(H₂O)_{y}. Examples of saccharide moieties include the D and L forms of glucose, fructose, xylose, arabinose, fucose, galactose, pyruvic acid, succinic acid, acetic, acid, galactose, 3,6-anhydrogalactose sulfate, galactose-4-sulfate, galactose-2-sulfate, galactose-2, 6-disulfate, mannose, glucuronic acid, mannuronic acid, guluronic acid, galactouronic acid, rhamnose, and so on. Preferably the polysaccharides used to make the MPM have molecular weights ranging from about 500 to 15,000,000 daltons, preferably 5,000 to 6,000,000, more preferably 25.000 to 1,000,000 daltons.

These polysaccharides are either added exogenously or produced by a microorganism. Examples of suitable anionic polysaccharides include galactans, galactomannans, glucomannans, polyuronic acids, and the like, which exhibit the requisite number of pendant hydrophilic groups. Suitable galactans are agar, agarose, kappa carageenan, iota carageenan, lambda carageenan, and the like. Examples of suitable galactomannans are locust bean gum and guar; examples of glucans are cellulose and derivatives thereof, starch and derivatives, dextrans, pullulan, beta 1,3-glucans, chitin, xanthan, tamarind and the like; examples of glucomannans are konjac; examples of polyuronic acids are algin, alginates, pectins; examples of heteropolysaccharides are gellan, welan, gum arabic, karaya gum, okra gum, aloe gum, gum tragacanth, gum ghatti quinceseed gum, psyllium, starch arabinogalactan and so on. Also suitable are dextran sulfate, heparin, pectin, sodium alginate, and mixtures thereof.

These polysaccharides may be further modified as taught in Aoki, T. T.; Araki & M. Kitamikado; 1990, Vibrio sp. AP-2. Eur. J. Biochem, 187, 461-465, provided it contains the requisite number of hydrophilic pendant groups. Also suitable for use in the compositions of the invention are chemically modified galactans, such as those taught in an article authored by K. B. Guiseley in Industrial Polysaccharides;Genetic Engineering, Structure/Property Relations and Applications, Edited by M. Yalpani, 1987, Elsevier Science Publishers. The Guiseley article teaches methods for the chemical modification of agar to obtain optimum gelling properties. In general, any modification of the galactans which does not affect the helical conformation (i.e. which is obtained via linkage of the 06 and 04 of galactose to the 02 of 3,6-anhydrogalactose) will preserve the gelling capability and is suitable for use in the compositions of the invention provided the requisite number of hydrophilic groups are present. The hydrophilic groups provide a polysaccharide which is water soluble. Many other polymers can be added before, during or after the fermentation process. They can be used to change 1) the functional properties of the MPMs, 2) the physical chemistry properties of the MPMs, 3) the aggregation of proteins, 4) the capacity to formulate or encapsulate various components from the various sectors like food, cosmetics, nutraceuticals and pharmaceuticals and 5) the biological activity of the MPMs. Examples of polymers like polyethylene glycol, polyehtyleneimine, polyesters, mono-, di-, or tri-block copolymers or any polymers helping the formation of colloid systems could be used to improve MPMs.

### Microorganisms

The bacteria used in the invention is R2C2 (Accession-Number 041202-3, National Microbiology Laboratory, Health Canada, 1015 Arlington Street, Winnipeg, Manitoba, Canada, R3E 3R2), INIX, ESI and/or K2. The process can further integrate a variety of other bacteria either alone or in combination like Bifidobacterium adolescentis, Bifidobacterium angulatum, Bifidobacterium animalis, Bifidobacterium asteroides, Bifidobacterium bifidum, Bifidobacterium boum, Bifidobacterium breve, Bifidobacterium catenulatum, Bifidobacterium choerinum, Bifidobacterium coryneforme, Bifidobacterium cuniculi, Bifidobacterium dentium, Bifidobacterium gallicum, Bifidobacterium gallinarum, Bifidobacterium indicum, Bifidobacterium infantis, Bifidobacterium longum, Bifidobacterium longum DJ010A, Bifidobacterium longum NCC2705, Bifidobacterium magnum, Bifidobacterium merycium, Bifidobacterium minimum, Bifidobacterium pseudocatenulatum, pseudolongum, Bifidobacterium ifidobacterium pseudolongum subsp. globosum, Bifidobacterium pllorum, Bifidobacterium ruminantium, Bifidobacterium saeculare, Bifidobacterium scardovii, Bifidobacterium subtile, ifidobacterium suis, Bifidobacterium thermacidophilum, Bifidobacterium thermacidophilum subsp. suis, Bifidobacterium thermophilum, Bifidobacterium urinalis, Lactobacillus, acetotolerans, Lactobacillus acidipiscis, Lactobacillus acidophilus, Lactobacillus agilis, Lactobacillus algidus, Lactobacillus alimentarius, Lactobacillus amylolyticus, Lactobacillus amylophilus, Lactobacillus amylovorus, Lactobacillus animalis, Lactobacillus arizonensis, Lactobacillus aviarius, Lactobacillus bifermentans, Lactobacillus brevis, Lactobacillus buchneri, Lactobacillus casei, Lactobacillus cellobiosus, Lactobacillus coleohominis, Lactobacillus collinoides, Lactobacillus coryniformis, Lactobacillus coryniformis subsp. coryniformis, Lactobacillus coryniformis subsp. torquens, Lactobacillus crispatus, Lactobacillus curvatus, Lactobacillus cypricasei, Lactobacillus delbrueckii, Lactobacillus delbrueckii subsp. bulgaricus, Lactobacillus delbrueckii subsp. delbrueckii, Lactobacillus delbrueckii subsp. lactis, Lactobacillus durianis, Lactobacillus equi, Lactobacillus farciminis, Lactobacillus ferintoshensis, Lactobacillus fermentum, Lactobacillus fornicalis, Lactobacillus fructivorans, *Lactobacillus frumenti, Lactobacillus fuchuensis, Lactobacillus gallinarum, Lactobacillus gasseri, Lactobacillus graminis, Lactobacillus hamsteri, Lactobacillus helveticus, Lactobacillus helveticus subsp. jugurti, Lactobacillus heterohiochii, Lactobacillus hilgardii, Lactobacillus homohiochii, Lactobacillus infestinalis, Lactobacillus japonicus, Lactobacillus jensenii, Lactobacillus johnsonii, Lactobacillus kefir, Lactobacillus kefiri, Lactobacillus kefiranofaciens, Lactobacillus kefirgranum, Lactobacillus kimchii, Lactobacillus kunkeei, Lactobacillus leichmannii, Lactobacillus letivazi, Lactobacillus lindneri, Lactobacillus malefermentans, Lactobacillus mali, Lactobacillus maltaromicus, Lactobacillus manihotivorans, Lactobacillus mindensis, Lactobacillus mucosae, Lactobacillus murinus, Lactobacillus nagelii, Lactobacillus oris, Lactobacillus panis, Lactobacillus pantheris, Lactobacillus parabuchneri, Lactobacillus paracasei, Lactobacillus paracasei subsp. paracasei, Lactobacillus paracasei subsp. tolerans, Lactobacillus parakefiri, Lactobacillus paralimentarius, Lactobacillus paraplantarum, Lactobacillus pentosus, Lactobacillus perolens, Lactobacillus plantarum, Lactobacillus pontis, Lactobacillus psittaci, Lactobacillus reuteri, Lactobacillus rhamnosus, Lactobacillus ruminis, Lactobacillus sakei, Lactobacillus sakei* L45, *Lactobacillus salivarrus, Lactobacillus salivarius subsp. salicinius, Lactobacillus salivarius subsp. salivarius, Lactobacillus sanfranciscensis, Lactobacillus sharpeae, Lactobacillus sp.* NGRI 0001, *Lactobacillus suebicus, Lactobacillus thermotolerans, Lactobacillus vaccinostercus, Lactobacillus vaginalis, Lactobacillus vermiforme, Lactobacillus versmoldensis, Lactobacillus zeae, Lactococcus garvieae, Lactococcus lactis, Lactococcus lactis subsp. cremoris, Lactococcus lactis subsp. hordniae, Lactococcus lactis subsp. lactis, Lactococcus lactis subsp. lactis bv. diacetylactis, Lactococcus piscium, Lactococcus plantarum, Lactococcus raffinolactis, Leuconostoc argenfinum, Leuconostoc carnosum, Leuconostoc citreum, Leuconostoc fallax, Leuconostoc ficulneum, Leuconostoc fructosum, Leuconostoc gasicomitatum, Leuconostoc gelidum, Leuconostoc inhae, Leuconostoc kimchii, Leuconosfoc lactis, Leuconostoc mesenteroides, Leuconostoc mesenteroides subsp. cremoris, Leuconostoc mesenteroides subsp. dextranicum, Leuconostoc mesenteroides subsp. mesenteroides, Leuconostoc mesenteroides subsp. mesenteroides* ATCC 8293, *Leuconostoc pseudomesenteroides, Propionibacterium acidipropionici, Propionibacterium acnes*, *Propionibacterium australiense, Proplonibactetium avidum, Propionibacterlum cyclohexanicum*, *Propionibacterium freudenreichii, Propionibacterium freudenreichii subsp*. *freudenreichii, Propionibacterium freudenreichii subsp*. *shermanii, Propionibacterium granulosum, Propionibacterium jensenii, Propionibacterium lymphophilum, Proplonibacterium microaerophilum, Propionibacterium propionicum, Propionibacterium thoenii, Saccharomyces delbrueckii, Saccharomyces cerevisiae*, *Saccharomyces unisporus, Saccharomyces globosus, Saccharomyces carisbergensis*, *Kluyveromyces fragilis, Kluyveromyces bulgaricus, Kluyveromyces lactis, Torula holmii, Candida tenuis*. The bacteria are preferably homolactic but can be heterolectic.

An MPM can use various genders and species and examples will be given to demonstrate that they modify functional properties of the MPMs like their hydration and emulsification capacities, their beneficial effects of health or their respective conservation times. MPMs generated with probiotic strain like *Lactobacillus plantarum* allows a better stimulation of the intestinal flora. MPMs generated with *Lactococcus lactis* allow the production of bacteriocin, NISIN, leading to an improved conservation time of the matrices. MPMs generated with *lactobacillus kefiranofaciens* or *L. ramnosus* 9595 allow a better positive overall stimulation of the immune system by their EPS, Murofushi et al. 1986. Immunopharmacology. Vol. 12. pp 29-35. MPM can prolong conservation of products in which it was added or incorporate. Yogurt containing MPM exhibited a better shelf life than the MPM-free yogurt. In addition, MPM can help maintaining survival of microorganisms for a prolonged period of time. Furthermore, MPM in yogurt serve as a stabilizing agent and replace either gelatine, pectine or corn starch.

In the process of the present invention, the culture of one bacteria can favorise the growth of a second or more microorganism in a sequential fermentation. A first fermentation of the lactic bacteria allows the growth of more demanding bacteria like bifidobacteria and propionibacteria.

The isolation of the bacterial strains (R2C2, K2, ES1) was performed on RCW agars as described by Kojima, S. et al., 1993, Biosei. Biotech. Blochem. Vol. 57, No. 1, pp: 119-120. Kefir grains were homogenized with a blender in an isotonic and sterile solution (tryptone 8,5 g/l + NaCl 1 g/l). This solution was used for RCW agar inoculation.

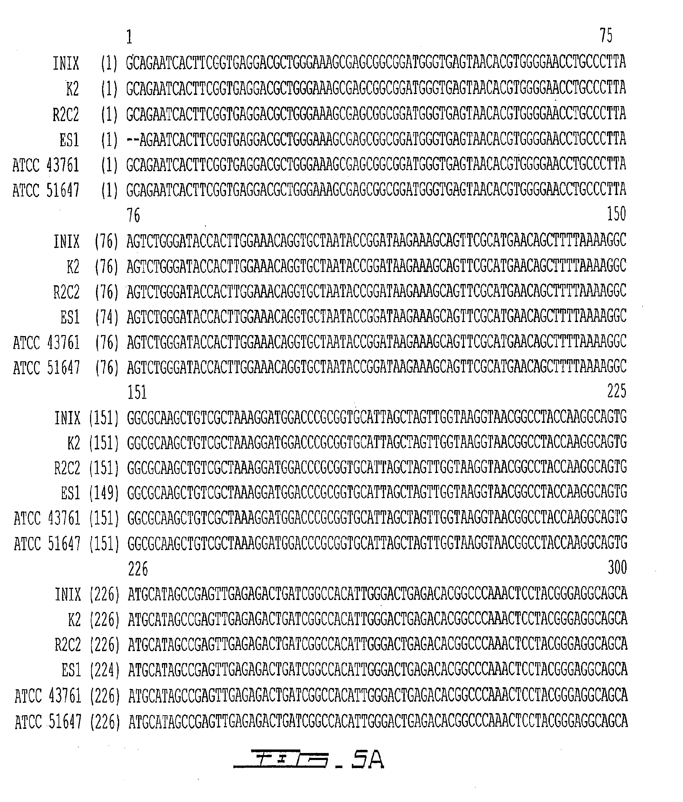

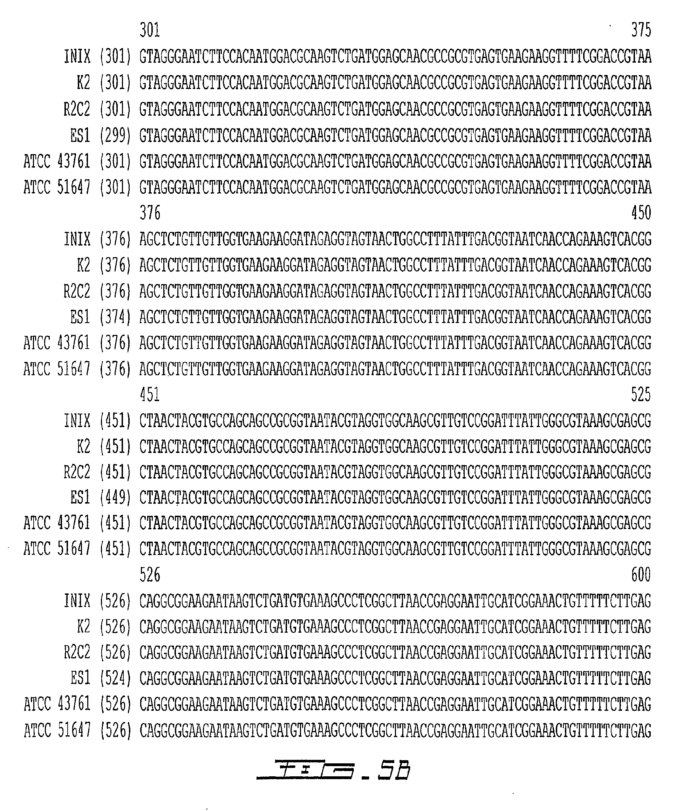

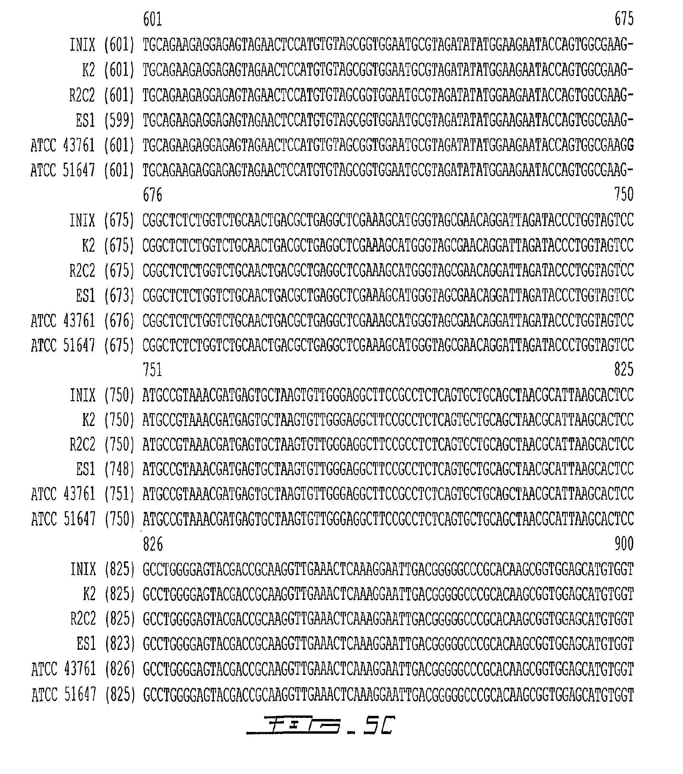

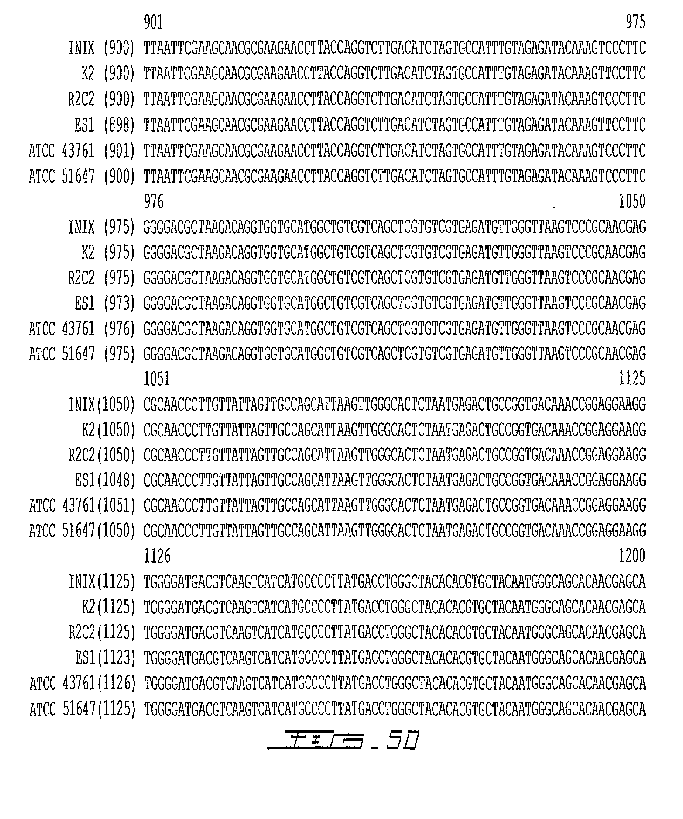

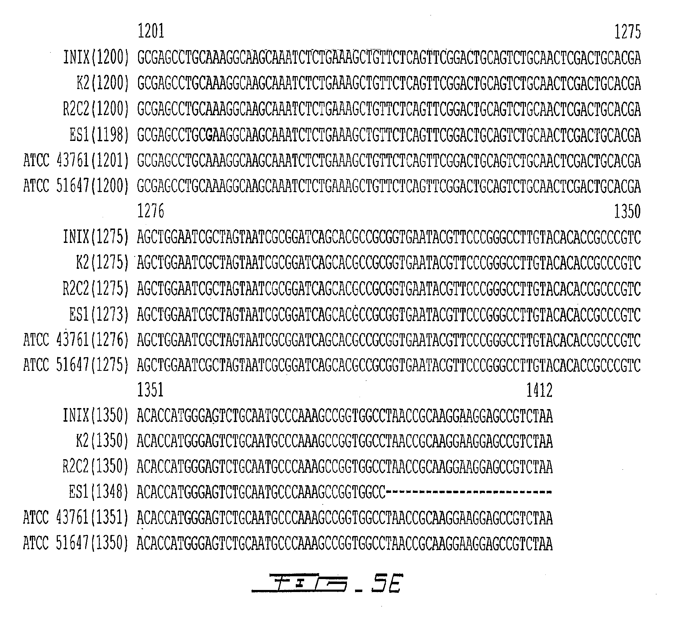

Different types of colonies were isolated. The selected strains are gram positives, non-mobile, catalase negatives and homofermetative strains. The strains are optionally anaerobic, not growing at 15°C and are having a samel physiology than the species described in Fujisawa et al., international journal of Systematic Bacteriology. Vol. 38. No. 1. pp:12-14. The strains were compared to the reference strain ATCC # 43761 for sugar fermentation pattern as illustrated at Table 1. Moreover, the strains were compared to the reference strains ATCC 43761 and 51647 for 16S homology as shown in Figs. 5A-B. The strains were compared to the reference stain ATCC 43761 for sugar fermentation pattern (as illustrated at Table 1) and to the reference strain ATCC 43761 and 51647 for 16S RNA homology (as illustrated at Table 2). The isolated strains were classified in the genus *Lactobacillus,* and the species *kefiranofaciens.*

**Table 1**

| **Fermentation profile of sugar from APA 50 CH and medium API 50 CHL** | | | | |
|---|---|---|---|---|
| **Substrates** | **R2C2** | **INIX** | **K2** | **ES1** |
| Glycerol | - | - | - | - |
| Erythritol | - | - | - | - |
| D-Arabinose | - | - | - | - |
| L-Arabinose | - | - | - | - |
| Ribose | - | - | - | - |
| D-Xylose | - | - | - | - |
| L-Xylose | - | - | - | - |
| Adonitol | - | - | - | - |
| β-Methyl glycoside | - | - | - | - |
| Galactose | +++ | +++ | +++ | +++ |
| D-Fructose | +++ | +++ | +++ | +++ |
| D-Mannose | ++ | +++ | +++ | +++ |
| L-Sorbose | - | - | - | - |
| Rhamnose | - | - | - | - |
| Dulcitol | - | - | - | - |
| Inositol | - | - | - | - |
| Mannitol | +++ | +++ | - | +++ |
| Sorbitol | - | - | - | - |
| α-Methyl-D-Mannoside | - | - | - | - |
| α-Methyl-D-Glucoside | - | - | - | - |
| N-acetyl glucosamine | ++ | ++ | ++ | ++ |
| Amygdaline | - | - | - | - |
| Arbutine | - | - | - | - |
| Esculine | ++ | - | +++ | +++ |
| Salicine | + | ++ | +++ | - |
| Cellobiose | - | - | +++ | - |
| Maltose | ++ | +++ | - | +++ |
| Lactose | +++ | +++ | +++ | +++ |
| Melbiose | - | - | - | - |
| Saccharose | +++ | ++ | - | +++ |
| Trehalose | +++ | +++ | - | +++ |
| Inuline | - | - | - | - |
| Melezitose | - | - | - | - |
| D-Raffinose | + | ++ | - | +++ |
| Starch | - | - | - | - |
| Glycogene | - | - | - | - |
| Xylitol | - | - | - | - |
| β-Gentibiose | - | - | +++ | - |
| D-Turanose | - | - | - | - |
| D-Lyxose | - | - | - | - |
| D-Tagarose | - | - | - | - |
| D-Fucose | - | - | - | - |
| L-Fucose | - | - | - | - |
| D-Arabitol | - | - | - | - |
| L-Arabitol | - | - | - | - |
| Gluconate | - | - | - | - |
| 2-ceto-gluconate | - | - | - | - |
| 5-ceto-gluconate | - | - | - | - |

### Factors influencing agglomeration

Salt is one factor for the retrieval of the MPM. In a preferred embodiment, CaCl₂ is used but could also be replaced by any salt that is known in the art to have an effect on protein agglomeration like sodium pyrophosphate. For the same purpose, parameters like pH, temperature, enzymatic hydrolysis can be varied to promote agglomeration of proteins to form a matrix.

### Food science

A need exists for a polysaccharide produced by a food-grade microorganism, having properties similar to or even superior to xanthan gum. Such an Exopolysaccharide (EPS) can either be added to the food product and the resulting product has to be labeled (but then the product is a so-called "friendly labeled" additive), or it can be produced *in situ* without the necessity of any labeling, because the microorganism is food-grade. The use of such microorganisms for the MPM production is preferred so the MPM produced offer either proteins characteristics and EPS properties. The MPMs can be used as a fat replacement agent, as a protein supplement, as a functional food product having a specific function (stimulation of the immune system, decreasing levels of triglyceride), as a bio-vehicle for ingredients, flavors, supplements, food additives, vitamins, etc.

### Cosmetic

The MPM combined in one matrix polysaccharides and proteins can be used in a wide variety of compositions, including foundation make-ups, skin lotions and creams, sunscreens, blushes, mascara, eye shadows, in addition to hair care products such as shampoos, conditioners, and the like. Suggested ranges of MPM are 0,01-95%, preferably 0,05-50%, more preferably 0,1-30% by weight of the total composition. The composition into which the MPM is incorporated can contain at least one surfactant, which may be an anionic, amphoteric, nonionic, cationic, or zwitterionic surfactant.

The MPM are suitable for use in foundation makeup or color cosmetics such as eye shadow, blush, concealer, or eyeliner compositions in the liquid, cream, solid, or stick form. Suitable compositions may be water-in-oil or oil-in-water emulsions, but are preferably oil-in-water emulsions. Such compositions generally comprise: 0,01-95% MPM, 0,5-95% water, 0,5-25% particulate matter, 0,01-20% surfactant, and 0,1-95% oil. In addition, these compositions may further contain ingredients selected from the group of humectants, preservatives, nonvolatile or volatile oils, gellants, and mixtures thereof.

### Nutraceuticals

The MPM of the present invention possesses the synergical sum of the physiological effects of Exopolysaccharides, whey proteins, bacteria, fermentation products that are parts of the MPM, and thus may be used in nutraceuticals.

MPMs have also multiple advantages in the field of probiotics. First, MPMs constitute a mean to produce probiotics at a low cost. During the MPMs retrieval, all bacteria in suspension are retrieved in the MPMs, which represents around 5% of the fermented volume and thus a concentration factor of 20. A fermented solution, containing 1x10⁹ bacteria generates a concentration of 2x10¹⁰ bacteria in the MPMs. Production of probiotics will at the same time lead to the retrieval of components having health benefits like proteins, peptides and fermentation by-products such as exopolysaccharide, vitamins, bacterial proteins, etc. MPMs constitute also a multifunctional vehicle for probiotics. MPMs allow the incorporation of hydrophobic or hydrophilic substances that an be used to protect, synergize and feed the probiotics. For instance, vitamin C, which is hydrophilic, helps maintaining viability of concentrated probiotics. Presence of certain exopolysaccharides (as for example oligogalactosaccharides) has a prebiotic effect (synergy) on the stimulation of the intestinal flora or the presence of vitamin E (hydrophobic), has protecting effect on the viability of the microorganism (antioxidant) and a nutritive effect (vitamin). In addition, because of its composition in proteins, MPMs are potentially capable of protecting the viability of probiotics. Finally, MPM can serve as a vehicle in different forms, humid, lyophilized or in compressed tablets. All forms constitute advantages in the field of probiotics. Humid MPMs are easy to formulate as shown in food formulations, cosmetics thus suggesting the same for the formulation of probiotics. Lyophilized MPMs, offer an important protection potential because of its content in proteins and the possibility of the incorporation of hydrophilic and hydrophobic protecting substances. Lyophilized MPMs are compressible without the need to add any excipients to form tablets which can be used for incorporation of probiotics or drugs.

### Pharmaceuticals

Several drugs may be formulated with the MPM and they may be delivered orally and topically.

A plurality of pharmaceutically related products and drugs or bioactive materials can be formulated with the MPM like small molecules of various classes (hydrophilic and hydrophobic), proteins, RNA, oligonucleotides, DNA, viruses, bacterias. Examples or types of bioactive materials that are used in the MPM and methods of the present invention include any pharmaceutical agents, including, but not limited to anti-inflammatory drugs, analgesics, anti-arthritic drugs, antispasmodics, antidepressants, antipsychotics, tranquilizers, antianxiety drugs, narcotic, antagonists, antiparkinsonism agents, cholinergic agonists, chemotherapeutic drugs, immunosuppressive agents, antiviral agents, antibiotic agents, appetite suppressants, antiemetics, anticholinergics, antihistaminics, antimigraine agents, coronary, cerebral or peripheral vasodilators, hormonal agents, contraceptives, antithrombotic agents, diuretics, antihypertensive agents, cardiovascular drugs, opioids, and the like.

Suitable bioactive materials also include therapeutic and prophylactic agents. These include, but are not limited to any therapeutically effective biological modifier. Such modifiers include, but are not limited to lipids, organics, proteins and peptides (synthetic and natural), peptide mimetics, hormones (peptides, steroid and corticosteroid), D and L amino acid polymers, oligosaccharides, polysaccharides, nucleotides, oligonucleotides and nucleic acids, including DNA and RNA, protein nucleic acid hybrids, small molecules and physiologically active analogs thereof. Further, the modifiers may be derived from natural sources or made by recombinant or synthetic means and include analogs, agonists and homologs. As used herein "protein" refers also to peptides and polypeptides. Such proteins include, but are not limited to enzymes, biopharmaceuticals, growth hormones, growth factors, insulin, monoclonal antibodies, interferons, interleukins and cytokins. Organics include, but are not limited to pharmaceutically active chemicals with amino, imino and guanidino groups. Suitable steroid hormones include, but are not limited to estrogen, progesterone, testosterone and physiologically active analogs thereof. Numerous steroid hormone analogs are known in the art and include, but are not limited to estradiol, SH-135 and tamoxifen. As used herein, "nucleic acids includes DNA, RNA and physiologically active analogs thereof. The nucleotides may encode single genes or may be any vector known in the art of recombinant DNA including, but not limited to, plasmids, retroviruses and adeno-associated viruses.

The MPMs described in the present invention have an advantage over conventional tablets pill in the above-described patients as it is a non-solid, creamy biodegradable vehicle that can be easily swallowed. Certain polysaccharides found in the MPMs, like kefiran products by *L*. *Kefiranofaciens,* are known to pass into blood circulation. These polysaccharides, peptides and bacteria found in the different MPM increase the absorption of some medicaments.

The following non-limiting examples further illustrate the invention and must not be contemplated as to limit the scope of the present invention.

### EXAMPLE 1

### Preparation of MPM

The preparation of a typical MPM is described in the following example.

Whey obtained from cheddar production is sterilized by filtration (0.22 µm). The sterilized whey is contained in a fermentation chamber at the time of inoculation with the R2C2 strain. A pre-culture is prepared to get a concentration of bacteria of 10⁸ to 10⁹ per ml of preculture medium. The inoculation is done with a volume of preculture medium (10⁸ R2C2/ml) corresponding to 1% and 15% but preferably 10% of the final volume of whey. The fermentation process is done at 37°C and at pH controlled at 5. The pH is controlled by the addition of NaOH. Agitation is maintained to a minimum to allow a uniform distribution but without causing an excessive aeration. The fermentation process is carried out at over a period of 16 to 36 hours depending of the characteristics needed. Following the fermentation process, between 0,1% and 1,5%, but preferably 1% of CaCl₂ (w/v) is added and the pH adjusted between 6,5 and 8, but preferably 7,5. The resulting MPMs is malleable, looks like a pudding of white creamy color with no noticeable taste or smell. Retrieval of the matrix is performed by centrifugation. Numerous centrifugal forces are appropriate depending on the functional characteristics desired. However, many tests indicated that a centrifugal force of 3500 RCF (Relative Centrifugal Force) is preferred for the production of a matrix with appropriate functional properties and that a centrifugal force between 3500 and 7476 RCF is also suitable.

An alternative process is using ES1, INIX, K2 or R2C2 and a comparative process *Lactobacillus helveticus* ATCC 10386, *Lactobacillus kefirgranum* ATCC 51647, *Lactobacillus ramnosus* ATCC 7469, *Lactobacillus* zeae ATCC 15820 or *Lactococcus lactis* ATCC 11454 and controlling culture conditions of pH and temperature at the controlling values shown in Table 2. An alternative process is adjusting initial pH at the controlling value of microorganism used as shown in Table 2.

**Table 2**

| **Parameters for different strains** | | |
|---|---|---|
| **Strains** | **Temperature (°C)** | **pH** |
| ES1 | 37 | 5 |
| INIX | 37 | 5 |
| K2 | 37 | 5 |
| R2C2 | 37 | 5 |
| ATCC10386 | 42 | 5,5 |
| ATCC 51647 | 30 | 5,5 |
| ATCC 7469 | 42 | 6 |
| ATCC 15820 | 42 | 6 |
| ATCC 11454 | 24 | 6 |

In another alternative process, the lactoserum is pasteurized before fermentation and the fermentated solution is pasteurized again before separation of the MPMs from the co-products. This alternative however is not used when active bacteria are needed in the final product.

In a further alternative process, a double inoculation is performed with R2C2 and *Lactococcus lactis* to get a co-culture. The two species can also be cultivated together for future innoculation.

In another comparative process, the strain used is *Propionibacterium acidipropionici* ATCC 4875, which produce propionic acid. Fermentation temperature is 30°C, pH is 7, and fermentation is performed for a period of 96 hours. Yeast extracts can be supplemented in proportions of 0.5 to 1% (w/v) to stimulate propionic acid production. When anaerobic bacteria are used, the process may use addition of gas like CO₂ or nitrogen to remove oxygen and/or increase CO₂ partial pressure.

### EXAMPLE 2

### Composition of spoonable salad dressing

The matrix is incorporated in the proportions as illustrated in Table 3, for producing a spoonable salad dressing. In this manner, a tasty, creamy and firm dressing similar to mayonnaise is obtained.

Thus, the formulation of the dressing is characterized by the fact that the dressing, when refrigerated at 4°C, keep all is properties. The matrix can replace egg yolks as emulsifiers and stabilizers in oil-water emulsions and the matrix can emulsified as much as its own volume of oil.

The salad dressing is prepared by adding sugar to MPM with agitation to prevent clumping, adding vinegar and corn syrup and stir with an Osterizer blender at maximum speed for 30 seconds, adding corn oil rapidly to the blender jar and maintaining mixing for 1 minute and store salad dressing at 4°C for at least 24 hours.

**Table 3**

| **Composition of spoonable salad dressing** | |
|---|---|
| | % |
| MPM | 37.6 |
| Salad oil (corn) | 37.3 |
| Corn syrup | 3.7 |
| Sugar | 1.5 |

### EXAMPLE 3

### Composition of chocolate milk

A chocolate milk is produced by mixing milk and MPM with an ultraturrax homogenizer, adding dry ingredient, adding liquid ingredients until dry ingredients are completely in solution and refrigerating at 4°C for at least 24 hours. The ingredients are listed in Table 4.

**Table 4**

| **Composition of chocolate milk** | |
|---|---|
| | % |
| Milk | 53.5 |
| MPM | 40.8 |
| Sugar | 4.7 |
| Chocolate flavor | 0.2 |
| Cocoa | 0.1 |
| Dairy Enhancer | To suit 100% |

### EXAMPLE 4

### Composition of light butter

A light butter is produced by softening butter at ambient temperature, mixing butter and MPM, homogenize mixture by using ultraturrax homogenizer until smooth-mixture is reached and refrigerate at 4°C for at least 24 hours. The ingredients are listed in Table 5.

**Table 5**

| **Composition of light butter** | |
|---|---|
| | % |
| Butter | 50-85 |
| MPM | 15-50 |

### EXAMPLE 5

### Use of MPM as thickening agent in yogurt

Yogurt is a dairy product obtained through the fermentation of milk by specific bacterial strains converting part of the lactose into lactic acid such as *Lactobacillus bulgaricus* and *Streptococcus thermophilus.* The milk coagulates when a sufficient quantity of lactic acid is produced.

Since the virtues of yogurt are associated among other things with the bacteria's action in the intestine, their presence in a sufficient number is important. With respect to this, yogurt should by law, in several countries, contain at least 10 million bacteria per gram at the time it is marketed.

The composition standards stipulate that yogurt must contain not less than 9.5% non-fat milk solids and not less than 3.0% protein. It may also contain some ingredients that come from milk (either whole or skim milk powder, or concentrated evaporated milk), fruits, fruit juices or extracts, jams, cereals or any other flavouring, sweeteners, a quantity not exceeding 2.0% of texturizing agents (stabilizers, gelling, thickening or emulsifying agents), citric acid, food colouring and, in the case of yogurt with added fruit, fruit juices or extracts or jams, a preservative not exceeding 50 ppm.

Potential use of the MPM, as thickening agent in yogurt, may be possible to enhance texture and viscosity in replacement of carrageenan, pectin, gelatin and corn starch among others. The MPM can be of interest as a dairy ingredient in a dairy product since it is alow calorie ingredient.

### Comparative EXAMPLE 6

### MPM as a vehicle to preserve microorganisms alive

MPM can maintains bacteria in life for a long period of time. Three samples of MPM R2C2 (produced with three different centrifuge force: 1592, 3500 and 7476 RCF) were conserved at 4°C during 11 months. After that period of time, a small quantity of MPM was inoculated on RCW agar. The gels were incubated without oxygen at 37°C for 5 days. After incubation, the R2C2 strain was isolated from the samples. It was found that MPM can maintains bacteria in life for a prolonged period of time and therefore can be used as probiotic vehicle.

### Comparative EXAMPLE 7

### Production of MPM containing a probiotic strain

**Table 6**

| **Production parameters** | |
|---|---|
| Parameters | Lactobacillus rhamnosus 7469 |
| * bacterial count | 5,1 x 10⁸ b/ml |
| Lactic acid; t0 | 0,439 g/l |
| Lactic acid, End of fermentation | 1,67 g/l |
| Lactic acid in Residual Solution | 0,215 g/l |
| MPM | 15,82 g/kg |
| Total glucide, t0 | 59,25 g/l |
| Glucide at the end | 51,0 g/l |
| Glucide in MPM | 48,0 g/l |
| Lactose at T0 | 45,52 g/l |
| Lactose at the end | 39,68 g/l |
| Lactose in Residual solution | 42,40 g/l |
| Lactose in MPM | 20,70 g/kg |
| Yield of MPM | 28,69 g/l |
| % humidity | 84,67% |
| % organic matter | 9,01% |
| % minerals | 4,95% |

| | |
|---|---|
| * from the culture before retrieval of the MPMs. The count in the MPMs is 20 times higher. | |

### EXAMPLE 8

### Makeup production

The MPM as prepared from the method described at the Example 1 was used to prepare two formulations of makeup sticks as follows:

**Table 7**

| **Makeup formulations** | | |
|---|---|---|
| **Formula** | **Components** | **w/w %** |
| A | Sodium stearate | 7.56 |
| | Water | 43.77 |
| | Phenoxyethanol | 0.50 |
| | Propyl paraben | 0.10 |
| | Methyl paraben | 0.30 |
| | Butylene glycol | 13.04 |
| | Calcium chloride | 0.80 |
| | MPM | 0.80 |
| | PEG-20 methyl glucose sesquiisostearate | 3.49 |
| | | |
| B | Hydrogenated castor oil | 2.00 |
| | Isostearyl alcohol | 5.74 |
| | Titanium dioxide | 0.74 |
| | Iron oxide yellow | 1.05 |
| | Iron oxide red | 0.33 |
| | Iron oxide black | 0.13 |
| | Talc | 2.23 |
| | Dimethicone | 11.63 |
| | Titanium dioxide/trimethylolethane | 6.30 |

### EXAMPLE 9

### Preparation of a skin lotion

A skin lotion was made according to the following formula:

**Table 8**

| **Skin lotion formulation** | |
|---|---|
| Components | w/w % |
| MPM | 1.60 |
| Trisodium EDTA | 0.10 |
| Butylene glycol | 5.00 |
| Sorbitan stearate/sucrose cocoate | 6.00 |
| Methyl paraben | 0.25 |
| Ethyl paraben | 0.15 |
| Xanthan gum | 0.30 |
| Octyl methoxycinnamate | 7.50 |
| Octyl salicylate | 5.00 |
| Benzophenone-3 | 3.00 |
| C12-15 alkyl benzoate | 8.00 |
| Cetyl alcohol | 1.00 |
| Phenoxyethanol | 1.00 |
| Propyl paraben | 0.10 |
| Water | QS |

### EXAMPLE 9

### Encapsulation of trypan blue and fluorescein with MPMs

Marker molecules like trypan blue and fluorescein were encapsulated in MPMs at various concentrations. These formulations were used to perform *in vitro* and *in vivo* experiments to study pharmacokinetic parameters like absorption, bioavailability, distribution, metabolism, and excretion of MPM-based oral formulations. Solubility, stability, liberation were analyzed and compared to the free markers. MPMs were found to have high encapsulation capacity and allow an overall improvement of general pharmacokinetic parameters.

To evaluate the efficacy of a fluorescent probe (Fluorescein (Sigma, Canada)) to penetrate the bloodstream in complexation with a potential drug carrier, female Wistar rats aged 8 weeks were used. The average rat weight was 200 g and they were fed *ad libitum.* Fluorescein (5mg/Kg of body weight) was vortexed either in saline 0,9% or in MPM before gavage. 3 rats/group were fed 1ml of mixed solution via feeding needles at T0 (Ttime). Fluorescein was only present in the first gavage. Rats were gavaged twice a day at an interval of 4 hours. 300µl blood samples were collected at T1, T3,5. When possible, urine was collected before bleedings. Blood was centrifuged at 13000 rpm for 5 minutes. Plasma was collected. 20 µl of plasma were diluted in 2 ml of PBS pH 7,2. Readings were recorded in a spectrofluorometer Eclipse (Varian, Australia) at an excitation wavelength of 490nm and an emission of 514nm. Concentrations were determined against a standard curve of fluorescein. The data below were collected showing that in fluorescein is entering blood circulation slightly better than when formulated in saline and excreted better in animal gavaged with MPMs suggesting a better absorption of fluorescein.

**Table 9**

| **Fluorescein concentration in blood over time** | | |
|---|---|---|
| Time (hours) | 1h | 3h30 |
| | (ng/ml) | (ng/ml) |
| Saline 0,9% | 336,699 | 99,693 |
| MPM-R2C2 | 415,701 | 116,622 |
| MPM-Inix | 323,532 | 84,645 |

**Table 10**

| **Fluorescein concentration in urine over time** | | |
|---|---|---|
| Time (hours) | 1h | 3h30 |
| | (ug/ml) | (ug/ml) |
| Saline 0,9% | 96,3072 | 125,6508 |
| MPM-R2C2 | 176,49423 | 126,027 |
| MPM-Inix | 213,58755 | 125,0865 |

### EXAMPLE 10

### Conditions for industrial production of MPMs

The preculture medium is a medium composed of whey permeate (62,5 g/l) containing 10g/l yeast extract (1%). Sterilized water is used to reconstitute powder whey. Powder of whey permeat is added along with the yeast extract. The medium is pasteurised at 90°C for 30 minutes avoiding the caramel-formation phenomenon and allowing a better ferment growth.

The preculture is generated in fermentor in the following conditions: 39°C, initial pH of 5 is controlled at 4,3 during fermentation, minimal agitation (50 RPM) for 18 to 20 hours with a ratio of initial inoculation between 1 to 15%, but preferably 10% (10⁸ bacteria/ml). The inoculation is performed from frozen ferments. The same conditions are used to ferment whey in order to produce the MPMs, but 1 % CaCl₂ is added to cruce whey, just before the pH adjustment at 5 witch HCl. This minimizes the risks of contamination and allows to bring the starting pH close the optimal growth of the bacterial strain used. Once the pH adjusted, whey is pasteurized in the fermentor at 70°C for 40 minutes, temperature is brought to 39 °C and whey is inoculated with 0,5 to 5%, but preferably 2,5% of preculture as defined previously. The fermentation of whey lasts 16 hours with the R2C2 strain. The pH is controlled by adding NaOH. Agitation is maintained to a minimal to allow a good distribution but without causing an excessive aeration. Reajustement of the pH at 7,5 and the retrieval of the MPMs is performed with an industrial clarifier Westfalia model NA-7. The yields obtained depend on the desired firmness and vary between 30 to 50 g/l of fermented solution. The MPM retrieval trigger the recuperation practically of all bacteria found in suspension.

### EXAMPLE 11

### Inoculation with frozen ferment in whey for MPM production

The MPM was prepared in the same conditions as in the Example 10 except that the fermentation was inoculated without preculture but directly in whey with a frozen ferment. The growth of the ferment is slower and requires a longer fermentation time (24 hours). The quality and the yield of retrieved MPM in those conditions is comparable to the use of ferment by picking in a preculture as described previously.

### EXAMPLE 12

### Storage of R2C2 preculture for the MPM production

The preculture of R2C2 as described in the Example 10 can be refrigerated and stored for 2 days without any noticeable alteration. A longer period of refrigeration of 72 hours triggers the apparition of latent phase of the ferment during a subsequent whey fermentation.

### EXAMPLE 13

### Pasteurization of the fermented whey

Fermented whey can be submitted to a thermal treatment of 65°C for 30 minutes in order to ensure the absence of viable contaminants. The pasteurization of the fermented solution has however to be avoided if ones want to preserve the beneficial effects of the probiotics when used in the fermentation process.

### EXAMPLE 14

### The use of a clarifier to retrieve the MPMs

Following a fermentation as described in the Example 10 of MPMs, an equipment Westfalia, model NA-7, was tested to continuously retrieve by means of nozzle or to retrieve by periodical discharges. The retrieval with nozzle generates a less dense and liquid MPM. This kind of MPM did not answer the needs for food formulations. In order to increase the density, a short residence time in the clarifier is preferable. This was accomplished by using the periodical discharges. The desired density of MPMs can be adjusted to satisfy the needs for food formulators for different products (butter and cream, etc.). The NA-7 is used at maximal speed of rotation with a flow of 330 L/h of fermented solution and discharged every 7 minutes intervals. The time of aperture of the bowl is adjusted to allow a partial appropriate discharge, eg. allowing neither the MPM accumulation inside the NA-7, nor a complete discharge of the bowl. The yields obtained are 40 to 45 g/L for MPMs having a spreading measurement of 8 to 10 cm while MPM obtained at a lower yield of 30 to 35 g/L for a spreading measurement of 3 cm. All agglomerates and microorganisms in suspension are retrieved.

### EXAMPLE 15

### Retrieval of MPM with a LAPX404

The LAPX404 machine from ALFA Laval is also utilized at a flow rate of 130 L/h, at a maximal rotation speed of 9500 RPM discharging every 7 minutes. The discharged volume with LAPX404 is partial but constant. In those conditions, density of the MPMs retrieved is appropriate for food formulations. The density can be adjusted by varying the discharges intervals and the flow rate. All agglomerates and microorganisms in suspension are retrieved.

### EXAMPLE 16

### MPM production from concentrated whey

In the same conditions as described in Example 10, concentrated whey (13% solid matter) is utilized as the fermentation start up solution. In those conditions, yields obtained for MPM production increased to 85 g per liter of fermented solution fermented with the R2C2 strain.

### EXAMPLE 17

### Protein recuperation by fermentation with specific microorganism

This example describes the use of *Lactobacillus kefirgranum* to help retrieving proteins in fermented solutions. *Lactobacillus kefirgranum* is inoculated from a lyophilised biomass in PL-salt medium as described: Tryptone peptone (casein) 1%, MgS04 0,02%, MnSO4 0,005%, Sodium acetate 0,2%, Tween 80 0,05%, Yeast Extract 1%, Powder of permeat of whey 62,5g/L. Complete with distilled water, adjust pH between 5,0 and 5,5 with HCl and autoclave at 121°C, 30 minutes. Keep at 4°C until use. Following the fermentation from 24 to 60 hours, preferably 40, at 30°C, the resulting culture can be filtrated or centrifuged or other treatment without any addition in order to retrieve the proteins and the bacteria forming agglomerates.

For the same application, *Lactobacillus kefirgranum* can be cultured in Rogosa Cheese Whey (RCW) medium prepared as follows: RCW is prepared from the Rogosa S1 Broth (Difco # 0478-17-4) and prepared according to the manufacturer's protocols except that distilled water is replaced by whey permeate which is prepared from a powder of whey permeate (62.5 g/L) and the proteins are denatured thermically (Sterilization 121°C for 15 minutes) and fractionated by filtration before use. *Lactobacillus kefiranofaciens* is grown at 30°C for 24 to 60 hours, preferably 40, to allow the retrieval of bacteria and part of the proteins. The culture can be filtered or centrifuged without any other treatment or addition to retrieve the proteins and the bacteria forming agglomerates.

Also, a 400-ml culture in PL-salt, for 24 hours of fermentation at 30°C, is used to inoculate 10 litres of sterile whey. Fermentation is controlled at pH 5,5, 30°C for 24 hours and the fermented solution is centrifuged without any other treatment or addition to retrieve the proteins and the bacteria forming agglomerates. The product retrieved is a MPM at pH 5,5. Another application is to adjust the pH of the fermented solution between 5,5 to 8, preferably at 7,5, before the retrieval of the agglomerates. Finally, another application is to adjust CaCl₂ between 0,1 and 1,5 %, preferably at 1 %, to the fermented solution before adjusting the pH between 5,5 and 8, preferably at 7,5, and to retrieve the agglomerates.

### EXAMPLE 18

### MPM as a flavor enhancing agent

In a panel of tasting, the majority of people concluded that the product containing the MPMs had an enhanced flavor. This was reported in butter (the salty taste) and in chocolate prepared drinks (the chocolate taste). Thus, MPM can be used to help increase the flavor of certain product.

### EXAMPLE 19

### Anti-inflammatory effect of MPMs (Reduction of TNF-α in blood cells)

Female Wistar rats weighing 150 g and fed *ad libitum* were submitted to 3 sessions of gavage (each session lasted a week for 7 repeated gavages) with a week of rest between each session. Blood samples were collected during and the course of the experiment and the rats sacrificed 2 h after the last gavage for a total of 21 gavages. The samples were harvested (blood cells), on dry ice, and frozen at -80°C. RNA was isolated using TRIZOL reagent (Gibco) as per manufacturers specifications. Ten micrograms of total RNA was reverse transcribed using Superscript RT (Gibco), 500ng oligodT primers (Gibco), and 250ng Random Hexamer primers (Gibco) for 20 minutes at room temperature, followed by 2 hours at 424°C.

TNF-α was amplified from 2ul of reverse transcription reaction using Titanium PCR kit (Clontech). Amplification primers were as follows: Rat TNF-α forward: CCCAACAA GGAGGAGAGTTCCC (SEQ ID NO:7) Rat TNF-α reverse: ATGACTCCAAAGTAGACCTGCCC (SEQ ID NO:8)

The PCR reaction was performed for 30 cycles using 100pmole for each primer, with an annealing temperature of 68°C. The PCR products were analysed on a 1.5% agarose gel. The data showed that the MPMs can reduce the level of TNF-α at the RNA level in blood cells after 7 days in the group of rats fed with MPMs in contrast to that observed in the groups fed with saline (- control), HMS90™ (a whey protein isolate sold by Immunotec), yogurt (Danone reduced in calories) therefore suggesting that an anti-infilammatory effect is taking place in blood cells.

### EXAMPLE 20

### Immunomodulatory effect of MPMs (production of IL-18)

Female Wistar rats weighing 150 g and fed *ad libitum* were submitted to 3 sessions of gavage (each session lasted a week for 7 repeated gavages). with a week of rest between each session. Blood samples were collected during and the course of the experiment and the rats sacrificed 2 h after the last gavage for a total of 21 gavages. The samples were harvested (blood cells), on dry ice, and frozen at -80 degrees. RNA was isolated using TRIZOL™ reagent (Gibco) as per manufacturers specifications. Ten micrograms of total RNA was reverse transcribed using Superscript RT (Gibco), 500ng oligodT primers (Gibco), and 250ng Random Hexamer primers (Gibco) for 20 minutes at room temperature, followed by 2 hours at 42°C.

IL-18 was amplified from 2ul of reverse transcription reaction using Titanium PCR kit (Clontech). Amplification primers were as follows:
IL-18 forward: ATGCCTGATATCGACCGAACA GCC (SEQ ID NO; 9)
IL-18 reverse: CAAATTCCATTTTGTTGTGTCCTG G (SEQ ID NO: 10)

The PCR reaction was performed for 30 cycles using 100pmole for each primer, with an annealing temperature of 684°C. The PCR products were analysed on a 1.5% agarose gel. The data showed that the MPMs increase.the levels of IL-18 at the RNA level in blood cells after 24 hours in the group of rats fed with MPMs in contrast to that observed in the groups fed with saline (- control), HMS90 (a whey protein isolate sold by Immunotec), yogurt (Danone reduced in calories) therefore suggesting a stimulation of the mucosal immunity.

### EXAMPLE 21

### Stimulation of PBMC in rats fed with MPMs

Female Wistar rats weighing 150 g and fed *ad libitum* were submitted to 3 sessions of gavage (each session lasted a week for 7 repeated gavages) with a week of rest between each session. Blood samples were collected during and the course of the experiment and the rats sacrificed 2 h after the last gavage for a total of 21 gavages. Blood samples were measured with Unopett (BD) to count the total peripheral monocular blood cells. The data are shown below and suggest that rats fed with MPMs have a tendency to see their count of PBMC to increase, almost doubled after 4 days post-gavage.

**Table 11**

| **PBMC count** | |
|---|---|
| Group test | PBMC count increase relative the saline |
| Saline | 1 |
| MPM | 1.8 |
| HMS 90 | 1.2 |
| Yogurt | 0.6 |
| Butter | 1.2 |
| Butter/MPM (40%MPM) | 1.6 |

### EXAMPLE 22

### Anti-triglyceridemia effect of MPMs

Female Wistar rats weighing 150 g and fed *ad libitum* were submitted to 3 sessions of gavage (each session lasted a week for 7 repeated gavages) with a week of rest between each session. Blood samples were collected during and the course of the experiment and the rats sacrificed 2 h after the last gavage for a total of 21 gavages. Serum were measured by the method of Wahlefeld (GPO-PAP) to determine the levels of circulating triglycerides. The data of table 12 below shows that MPMs affect the basal level of TG.

**Table 12**

| **Triglycerides levels after 24 hours and 3 days** | | |
|---|---|---|
| | TG after 24 hours mg/dl serum (SEM) | TG after 3 days mg/dl serum (SEM) |
| Saline | 62 (15) | 51 (4) |
| MPM | 33 (5) | 32(10) |
| HMS 90 | 31 (4) | 101 (15) |
| Yogurt | 118(38) | 76 (27) |
| Butter | 64 (30) | 128 (46) |
| Butter/MPM | 83 (9) | 156 (22) |

**Table 13**

| **Triglycerides levels after 24 hours, 16 days and 21 days** | | | |
|---|---|---|---|
| Test groups | TG after 24 hours mg/dl serum (SEM) | TG after 16 days mg/dl serum (SEM) | TG after 21 days mg/dl serum (SEM) |
| Saline | 71(14) | 80(19) | 43(14) |
| MPM | 50(15) | 52(1) | 35(1) |
| HMS 90 | 56(6) | 102(34) | 92(21) |
| Yogurt | 57(4) | 56(13) | 99(24) |

### EXAMPLE 23

### Analysis of the amino acid content of MPMs

MPMs were analyzed by Bodycote Canada in order to determine the comparative amino acid content of various whey protein-based product. WPH917 is a high quality whey protein hydrolysate produced by a controlled enzyme treatment of whey protein which provides amino acids, peptides, and polypeptides. Power Pro80 is 80% whey protein concentrate produced by an ultrafiltration process that concentrates native whey proteins.

**Table 14**

| **Analysis of the amino acid content** | | | |
|---|---|---|---|
| Amino acid | MPM (batch 15) | WPH 917 | PowerPro 80 |
| Glutamic Acid | 22,92 | 18,3 | 10,1 |
| Alanine | 5,73 | 5,2 | 4,1 |
| Arginine | 4,01 | 3 | 1,9 |
| Cystine | 10,32 | 2,9 | 2 |
| Glycine | 13,61 | 2,3 | 1,5 |
| Histidine | 11,17 | 1,9 | 1,6 |
| Isoleucine | 6,45 | 5,5 | 5,1 |
| Leucine | 11,89 | 14,2 | 9 |
| Serine | 7,02 | 5 | 3,9 |
| Thryptophane | Not determined | 2,3 | 1,4 |

### EXAMPLE 24

### Content analysis of the MPMs

MPMs were analyzed by Bodycote Canada in order to determine the overall content of various whey protein-based products. WPH917 is a high quality whey protein hydrolysate produced by a controlled enzyme treatment of whey protein which provides amino acids, peptides, and polypeptides.

**Table 15**

| **Content analysis** | | | |
|---|---|---|---|
| Content | Methods | MPM %(g/100g) | WPH917 %(g/100g) |
| Humidity | AC-HUM 04 | 80 | 4 |
| Proteins | AC-PRO01AOAC | 8 | 89 |
| Ashes | AC-CEN01AOAC | 6 | 3.1 |
| Carbohydrates | AC-SUB01AOAC | 5 | Not determined |
| Lactose | AC-LAB01AOAC | 2.5 | 0.3 |
| Fat | AC-GRA 01 | 1.3 | 3.5 |
| Minerals (Na, Ca, K) | SAA | 0.1, 1.8, 0.2 | 1.3,0.1,002 |

### EXAMPLE 25

### Resulting biological effect of pasteutized MPM-INIX on Gluthathione

Female Wistar rats weighing 150 g and fed *ad libitum* were submitted to 3 sessions of gavage (each session lasted a week for 7 repeated gavages) with a week of rest between each session. Blood samples were collected during and the course of the experiment and the rats sacrificed 2 h after the last gavage for a total of 21 gavages. Blood samples were measured after 3 weeks according to a method modified from Anderson designed to measure glutathione levels in plasma.

**Table 16**

| **GSH levels in rats** | |
|---|---|
| Test groups | levels of GSH In ug GSH/ml plasma (+/-SD) |
| Saline | 0.05 (0.05) |
| MPM 1* | 0.08 (0.08) |
| MPM** | 1.08 (0.31) |

| | |
|---|---|
| * Plain MPM produced with R2C2 ** Pasteurized MPM produced with INIX | |

### EXAMPLE 26

### Solubilization of pyrene using MPM

50 µM pyrene stock solution in acetone is prepared and 20 µL of the stock solution is brought into borosilicate tubes (10 x 13 mm). The tubes are standing at room temperature in a fumehood for 20-30 minutes until complete evaporation of acetone. 2.0 ml of MPM dissolved in 0,1 M phosphate buffered saline (PBS) at pH 7,2 are inserted into the borosilicate tubes. The concentration of MPM used is inside the range of 0,001 to 1,0% (w/v). Let the solution stand at room temperature, protected from the light and fluorescence readings are performed after 24, 48 and 69 h of incubation. Fluorescence reading are performed on a Varian Cary Eclipse at 37°C with an excitation wavelength set at 340 nm and emission scan from 350 to 600 nm are recorded. The degree of solubilization of pyrene is measured by plotting the I₃/I₃(aqueous) of the compound studied as a function of the concentration of the compound. The inflexion point of the plot correspond to the critical micellar concentration value. The data of Table 17 show the ratio of fluorescence intensity of peak I₃ over intensity of peak I₃ from PBS solution as a function of the concentration of MPM and P85.

**Table 17**

| **ratio of fluorescence intensity of peak I3 over intensity of peak I3 from PBS solution** | | |
|---|---|---|
| | P85 | MPM lot 78 |
| 0.001% | 1,107 | 1,123 |
| 0.01% | 1,168 | 1,328 |
| 0.1% | 2,564 | 2,416 |
| 1% | 8,813 | 3,687 |

The data of Table 18 show the ratio of fluorescence intensity of peak Iₑ over intensity of peak Iₑ from PBS solution as a function of the concentration of MPM and P85. The data show that MPMs promote the formation of excimers (hydrophobic microdomains).

**TabLe 18**

| **ratio of fluorescence intensity of peak le over intensity of peak le from PBS solution** | | |
|---|---|---|
| | P85 | MPM lot 78 |
| 0.001% | 0,9619 | 1.567 |
| 0.01% | 1,233 | 4,834 |
| 0.1% | 2,443 | 30,88 |
| 1% | 4,829 | 25,24 |

### EXAMPLE 27

### Incorporation of MPM in body lotion

In order to validate the functional properties of the MPMs in a cosmetic formulation, a test was done to formulate a commercial body lotion. The commercial product was a lotion made out goat milk containing 0.3% goat milk extract. The MPM was incorporated in a 10% (w/w). The vessel was closed before mixing the component and agitated by hand and stored at room temperature. After 24 hours, the resulting mixture exhibited fines particles on the vessel walls and was more liquid than the original commercial body lotion. However, the resulting mixture did not show signs of degradation or separation of emulsion and the smell remained similar to the commercial product. Viscosity of MPMs was evaluated using spreading techniques according to the principle of consistometry USDA (Adams & Birdsall, 1946) allowing the measurement, in cm, of the distance of spreading of a semi-fluid food performed on a plate equipped with a series of concentrated circles In predetermined and standardized time. The resulting mixture of body lotion + MPM recorded 13 cm on the spreading scale but get back to its original spreading e.g. 8cm over a period of 3 weeks. The data indicated that the addition of 10% de MPM triggered a liquefaction of the matrix. In terms of shelf-life preservation the resulting mixture was found stable and not contaminated for a period of 3 weeks with the same particle in suspension with the same characteristic smell than the original product.

### EXAMPLE 28

### Light butter

Two types of light butter were prepared containing 25 and 40% MPM, respectively. In general, the MPM-butter is creamier at room temperature, solidifies less at 4°C, and exhibit a texture which is more breakable than regular butter. It is important to note that the salty taste of butter was enhanced by the MPMs allowing a reduction of the usual amount of salt (NaCl) in the butter by 33 to 50%. The butter containing 25 % MPM is useable for frying and turn brown like plain butter while the 40% butter does not allow frying and is used only as a spread. Also, the conservation time of MPM-butter was acceptable for 45 days and the MPM-containg butter could be frozen without any change of organoleptic properties.

### EXAMPLE 29

### MPM in whipped cream

Light cream formulations were prepared from 40% cream, milk and MPM to obtain a cream with 21% fat that exhibit a nice and thick texture that can be Whipped. The resulting whipped cream is firm and can be stored for 15 days in the fridge without alteration. The odor and taste are similar than the plain cream. The recipe is as follows: 200 ml of cream at 40%, 85 ml milk, 95 g of MPM and sugar and /or aroma.

### EXAMPLE 30

### Mayonnaise and salad dressing

The same type of MPM used previously was used to prepare Mayonnaise and salad dressing according to the following formula:

**Table 19**

| **Salad dressing preparation** | |
|---|---|
| Ingredients : | Amount (grams) |
| MPM | 19.6 |
| oil | 19.4 |
| Corn syrup | 1.9 |
| sugar | 0.8 |
| aroma | To taste |
| spice | To taste |
| vinegar | 10.3 |

### EXAMPLE 31

### Chocolate drink

The same type of MPM used previously was used to prepare chocolate drinks according to the following formula:

**Table 20**

| **Chocolate drink preparation** | |
|---|---|
| Ingredient | Amount in grams |
| MPM | 60 |
| milk | 100 ml |
| cacao | 0.16 |
| sugar | 7 |
| Chocolate aroma | 0.25 |
| Flavor enhancer | To be adjusted |

This formulation allows the enrichment of milk of 3,6 g of proteins which is practically doubled as compared to plain milk. The MPM gives a better viscosity and a creamier texture which is appreciated in mouth. MPMs were also added to a weight control drink (Slim fast™) and the resulting product was found to have a reduced taste of the soy proteins found in that drink.

### EXAMPLE 32

### Yogurt

Four attempts were made with the formulation of yogurt. (1) control with 3,25% milk and (3) attempts with 1% milk all supplemented with 3% skim milk powder. The steps for the preparation of yogurt included heating the milk at 82-85°C, agitatingh for 30 min, followed with a cooling step at 44-45°C. The culture (yogourmet, LYO-SAN Co) containing *L*. *Bulgaricus, S. Thermophilus* and *L. Acidophilus* were added to get 5 g per liter of milk, followed by an incubation at 45°C for 4 hours, without agitation.
Control test made from 3,25% milk.
Test 2: simultaneously heating of the milk and the MPM (100g/l) followed by the addition of the ferment.
Test 3: the milk is heated first followed by the addition of the MPM (100 g/l) and the ferment.
Test 4: addition of the MPM at the end of the production, at the same time than the addition of the fruits like in an industrial production.

Following the incubation, the product obtained by the Test 2 is comparable to the control test while test 3 and 4 did not allowed the coagulation. After 6 weeks of storage, the control test exhibits a significant drainage and was contaminated with yeast while test 2 did not show neither signs of destabilization nor microbiological deterioration since its preparation. Thus, MPM play a role of preservative on the formulation and helps to obtain very good and typical texture for a yogurt while reducing the amount fat in the final formulation without the addition of starch or other thickening agents.

### EXAMPLE 33

### The use of different strains affect the analytical profile of the resulting MPMs produced in batches of 10 litres

Concentration of various carbohydrates (glucids), lactic acid and the yields of MPM vary according to the strain used. Also, the composition of the fermented solution influence the composition of the matrix either directly and proportionally like in the case of the carbohydrates found in the aqueous and liquid phase of the matrix, or according to a concentrating effect like in the case of lactic acid that precipitates in part during the retrieval of the matrix.

**Table 21**

| **MPM analytical profile in function of different strains used to produce them** | | | | | |
|---|---|---|---|---|---|
| | **INIX** | **Lactobacillus rhamno 7469** * | **Lactobacillus zeae** * | **Lactobacillus kefirgranum** * | **Lactobacillus helveticus** * |
| *bacterial count | 2,47 x 10⁸b/ml | 5,1 x 10⁸b/ml | 1,34 x 10⁸b/ml | Not determined | 6,77 x 10⁷b/ml |
| Lactic acid ; t0 | 0,211g/L | 0,439g/L | 0,143 g/L | 0,234 g/L | 0,157 g/L |
| Lactic acid, End of fermentation | 2,414g/L | 1,67g/L | 0,821 g/L | 2,22 g/L | 3,5 g/L |
| Lactic acid in Residual Solution | 1,122 g/L | 0,215 g/L | 0,384 g/L | 1,11 g/L | 1,53 g/L |
| **MPM** | 9,57 g/kg | 15,82 g/kg | 9,85 g/kg | 2,94 g/kg | 5,23 g/kg |
| Total glucide, t0 | 45,0 g/L | 59,25 g/L | 41,30 g/L | 54,22 g/L | 47,06 g/L |
| Glucide at the end | 41,25 g/L | 51,0 g/L | 55,01 g/L | 51,55 g/L | 42,67 g/L |
| Glucide in Residual solution | 42,0 g/L | 48,0 g/L | 53,86 g/L | 46,54 g/L | 54,24 g/L |
| Glucide in MPM | 36,50 g/kg | 29,35g/kg | 39,86g/kg | 38,593g/kg | 41,197g/kg |
| **Lactose at T0** | 47,95 g/L | 45,52 g/L | 53,46 g/L | 50,99 g/L | 52,58 g/L |
| Lactose at the end | 42,95 g/L | 39,68 g/L | 51,74 g/L | 47,28 g/L | 42,65 g/L |
| Lactose in Residual solution | 42,57 g/L | 42,40 q/L | 47,31 g/L | 44,92 g/L | 41,55 g/L |

| | | | | | |
|---|---|---|---|---|---|
| * comparative | | | | | |

**Table 21**

| **MPM analytical profile in function of different strains used to produce them** | | | | | |
|---|---|---|---|---|---|
| | **INIX** | **Lactobacillus rhamno 7469** ** | **Lactobacillus zeae** ** | **Lactobacillus kefirgranum** ** | **Lactobacillus helveticus** ** |
| Lactose in MPM | 24,78g/kg | 20,70g/kg | 27,70g/kg | 37,02g/kg | 35,17g/kg |
| **GALACTOSE at T0** | 0 | 0 | 0 | 0,13 g/L | 0 |
| Galac. At the end | 0,75 g/L | 0 | 0 | 0,75 g/L | 2,51 g/L |
| Galac. in Residual solution | 0,70 g/L | 0 | 0 | 0,36 g/L | 2,02 g/L |
| MPM | 1,72g/kg | 0 | 0 | 1,55g/kg | 2,22g/kg |
| **GLUCOSE at T0** | 0 | 0 | 0 | 0 | 0 |
| Glucose at the end | 0 | 0 | 0 | 0,49 g/L | 1,49 g/L |
| Glucose in Residual solution | 0 | 0 | 0 | 0 | 0,88 g/L |
| MPM | 0 | 0 | 0 | 1,48 g/kg | 1,74 g/kg |
| **Yield of MPM** | 41,2g/L | 28,69 g/L | 27,5 g/L | 40,6 g/L | 40,12 g/L |
| % humidity | 85,98% | 84,67% | 85,23% | 85,11% | |
| **% organic matter** | 9,49% | 9,01% | 8,66% | 9,87% | |
| **% minerals** | 4,53% | 4,95% | 6,12% | 5,03% | |

| | | | | | |
|---|---|---|---|---|---|
| * from the culture before retrieval of the MPMs. The count in the MPMs is 5 times higher. ** comparative | | | | | |

### EXAMPLE 34

### Characteristics of various MPMs produced from different strains

The use of different strains during the fermentation process affect texture, taste, and smell of resulting MPMs as shown in Table 22.

**Table 22**

| **Characteristics of MPM produced from different strains** | | | | | |
|---|---|---|---|---|---|
| **Parameters** | **Lactococcuslactis †** | **Lactobacillus helveticus †** | **INIX** | **Lactobacillus rhamnosus †** | **R2C2** |
| **pH** | 5.69 | 6.99 | 6.58 | 5.06 | 6,26 |
| **Color** | 7039-12 | 7042-22 | 7042-12 | 7039-12 | parsnip |
| **taste** | Acre | Acre | Acre | nd | Neutral |
| **smell** | Yogurt plain | Condensed milk | Algeas | Strong whey | Cooked cauliflower |
| **Appearence** | synerisis important | Nice homogenous texture | Nice soft and viscous texture | bubbles agglomerates | Heterogenous agglomerates |
| **%synerisis** | 0.07 | 0.08 | 0.1 | 0 | 0,23 |
| **Drainage** | nul | nul | nul | nul | nul |
| ***Spreading** | 1.25 | 2 | 2 | 5.5 | 0 |
| **** Emulsifying activity** | Mayonnaise | Vinaigrette | Vinaigrette | Vinaigrette | Vinaigrette |

| | | | | | |
|---|---|---|---|---|---|
| * Viscosity of MPMs was evaluated using spreading techniques according to the principle of consistometry USDA (Adams & Birdsall, 1946) allowing the mesureament, in cm, of the distance of spreading of a semi-fluid food performed on a plate equiped with a series of concentrated circles in predetermined and standadized time. ** The emulsifying activity is evaluated by means of model system representing a good capacity for the formation of a vinaigrette and of and of mayonnaise. † comparative | | | | | |

### EXAMPLE 35

### Characterization and composition of the MPMs produced in 10-litres in fermentors by the R2C2 strain.

**Table 33**

| **Characteristics of the MPMs vary according to the growth rate of the strain** | | | | | |
|---|---|---|---|---|---|
| **Parameters** | **Sample 1** | **Sample 2** | **Sample 7** | **Sample 8** | **Sample 7 pasteurized** |
| **pH** | nd | nd | 7.17 | 7.21 | 7.06 |
| **Color** | 7042-22 | 7042-22 | 7039-12 | 7039-12 | 7041-22 |
| **Taste** | bitter | bitter | bitter | bitter | nd |
| **Odor** | Cooked cauliflower | Cooked cauliflower | Cooked cauliflower | Cooked cauliflower | Algae |
| **Appearence** | Firm, no synerisis | Yogurt-like synerisis | homogenous soft | homogenous soft | homogenous soft Yogurt-like |
| **%synerisis** | 0 | 0.01 | 0.1 | 0 | 0 |
| **Drainage** | nil | nil | nil | nil | nil |
| **Spreading** | nd | nd | 8.25 | plus de 12 | 6.5 |
| **Emulsifying activity** | Vinaigrette | Vinaigrette | Vinaigrette | Vinaigrette | Vinaigrette |
| Bacterial count in MPM | 2 x 10⁹ b/g | 1,4x 10⁹ b/g | 5 x 10⁸ b/g | 6,7 x 10⁸ b/g | 5 x 10⁸ b/g |
| Lactic Acid | 16,39 mg/g | 24,38 mg/g | 7,02 mg/g | 5,61 mg/g | 7,02 mg/g |
| Total sugars | 15,70g/kg | 18,30 g/kg | 45,19g/kg | 43,15g/kg | 45,19g/kg |
| **Lactose** | 0 | 0 | 49,88g/kg | 48,57g/kg | 49,88g/kg |
| **Galactose** | 14,9g/kg | 18,1 g/kg | 0,66 g/kg | 0,77 g/kg | 0,66 g/kg |
| **Yield** | 27,32 g/L | 33,85 g/L | 41,10 g/L | 44,10 g/L | 41,10 g/L |
| **% humidity** | 82,26% | 84,82% | 86,05% | 85,86% | 86,05% |
| **% organic matter** | 10,92% | 9,37% | 9,01% | 9,86% | 9,01% |
| **% minerals** | 6,83% | 5,82% | 5,08% | 4,28% | 5,08% |

| | | | | | |
|---|---|---|---|---|---|
| Legend Nd, not determined 7039-12 white asparagus, off-white with greenish tendency. 7041-12 whiter and less green than 7039-12 (canevas) 7042-12 cauliflower, more beige than white 7042-22 White but with orange background | | | | | |

### Comparative EXAMPLE 36

### Production of MPMs containing proteins from plant origin

The preculture medium is a medium composed of de whey permeat (62,5 g/l) containing 10g/l yeast extract (1%). Sterilized water is used to reconstitute powder whey. Powder of whey permeat is added along with the yeast extract. The medium is pasteurised at 90°C for 30 minutes avoiding the caramel-formation phenomenon and allowing a better ferment growth.

The preculture is generated in fermentor in the following conditions: 39°C, initial pH of 5 is controlled at 4,3 during fermentation, minimal agitation (50 RPM) for 18 to 20 hours with a ratio of initial inoculation between 1 to 15%, but preferably 10% (10⁸ bacteria/ml). The inoculation is performed from frozen ferments. The same conditions are used to ferment whey (supplemented with commercial soy proteins 1 to 10% w/v) in order to produce the MPMs, but 1% CaCl₂ is added to crude whey, just before the pH adjustment at 5 with HCl. This minimizes the risks of contamination and brings the starting pH close the optimal growth of the bacterial strain used. Once the pH adjusted, whey is pasteurized in the fermentor at 70°C for 40 minutes, temperature is brought to 39°C and whey is inoculated with 0.5 to 5%, but preferably 2,5% of preculture as defined previously. The fermentation of whey lasts 16 hours with the R2C2 strain. The pH is controlled by adding NaOH. Agitation is maintained to a minimum to allow a uniform distribution without causing an excessive aeration. Readjustment of the pH at 7,5 and the retrieval of the MPMs is performed with a, industrial clarifier Westfalia model NA-7. The yields obtained depend on the desired firmness and vary between 30 to 50 g/l of fermented solution. The MPM retrieval trigger the recuperation practically of all bacteria found in suspension.

### EXAMPLE 37

### Formulation of 5-Fluoro Uracile with the MPMs

MPMs can be used to formulate various bioactive materials. In this example 5-Fluoro Uracile was formulated with the typical MPM described in the above examples and given orally by gavage to mice. The efficacy on this new formulation was compared to free 5FU and tested in animal models in which colon cancer cells were implanted or in which colon cancer was chemically inducted. The finding was that the 5FU/MPM formulation was improving the therapeutic index of 5FU as shown by reduction of tumor growth.

### EXAMPLE 38

### Basic formulation of a firm yogurt

1. Add 3 to 5% of mild solids to the milk
2. Heat milk to 85°C and maintain for 30 minutes
3. Reach ebullition and add 0.5% of gelatine, pectine or corn starch to the milk
4. Cool milk to 44-46°C and add bacterial strains converting part of the lactose into lactic acid such as *Lactobacillus bulgaricus* and *Streptococcus thermophilus*
5. Add 20% of MPM
6. Place in containers and incubate between 40-46°C until 80°D is reach, for 4-6 hours
7. Refrigerate at 4°C for at least 24 hours.

### EXAMPLE 39

### Composition of vanilla pudding

A vanilla pudding is produced by mixing milk and MPM, adding dry ingredient to the milk-MPM mixture in mixing well, adding slowly oil to the mixture by using ultra-turrax homogenizer, cooking between 60-70°C until the mixture thickens and refrigerating at 4°C for at least 24 hours. The ingredients are listed in Table 34.

**Table 34**

| **Composition of vanilla pudding** | |
|---|---|
| | % |
| MPM | 50 |
| Milk | 36.1 |
| Sugar | 14.9 |
| Vegetal oil | 3.3 |
| Na₂HPO₄ | 0.2 |
| Vanilla | 0.5 |

## Claims

1. A process for manufacturing a malleable protein matrix, said process comprising the steps of:
a) fermenting a whey protein solution with bacteries in a medium;
b) agglomerating proteins from the protein solution of step a); and
c) isolating agglomerated proteins from supernatant;
**characterised in that** said bacteries comprises at least one bacteria selected from R2C2, INIX, ES1 and K2.

2. The process of claim 1, wherein said fermenting step is promoted by co-culturing at least two microorganisms simultaneously or successively.

3. The process of claim 1, wherein said process further comprises a step between steps a) and b) for addition of a polysaccharide.

4. The process of claim 1, wherein said process further comprises a step between steps b) and c) for addition of a polysaccharide.

5. The process of any one of claims 1 to 4, wherein agglomeration of fermented proteins is effected by at least one method selected from the group consisting of salt addition, pH modulation, thermal treatment, proteolytic enzymes addition and flocculent addition.

6. The process of claim 2, further comprising a step of pasteurization of said proteins solution before step a).

7. The process of claim 6, wherein said step of pasteurization is followed by a step of sterilization.

8. The process of claim 5, wherein said flocculent is a bacterial flocculent.

9. The process of claim 8, wherein said bacterial flocculent is *L. Kefirgranum.*

10. The process of any one of claims 1 to 9, wherein separation of agglomerated proteins from supernatant is effected by a method selected from the group of centrifugation and filtration.

11. A malleable protein matrix obtainable by the process of claim 1.

12. The matrix of claim 11, further comprising a fermentation by-product of the fermentation of said solution containing said protein by said bacteria.

13. The matrix of claim 11, further comprising a peptide.

14. The matrix of claim 13, wherein said peptide comprises at least two amino acid residues.

15. The matrix of claim 13, wherein said peptide comprises more than one hundred amino acid residues.

16. The matrix of claim 12, wherein said fermentation by-product is a polysaccharide.

17. The matrix of claim 16, wherein said polysaccharide is selected from the group of exopolysaccharide and anionic polysaccharide.

18. The matrix of claim 16, wherein said polysaccharide contains at least four saccharide moieties.

19. The matrix of claim 18, wherein said saccharide moieties are selected from the group consisting of D and L forms of glucose, fructose, xylose, arabinose, fucose, galactose, pyruvic acid, succinic acid, acetic acid, 3,6-anhydrogalactose sulfate, galactose-4-sulfate, galactose-2-sulfate, galactose-2, 6-disulfate, mannose, glucuronic acid, mannuronic acid, guluronic acid, galactouronic acid, and rhamnose.

20. The matrix of claim 16, wherein said polysaccharide have molecular weight ranging from about 500 to about 15,000,000 daltons.

21. The matrix of claim 16, wherein said molecular weight is ranging from about 5,000 to 6,000,000 daltons.

22. The matrix of claim 16, wherein said molecular weight is ranging from about 25,000 to 1,000,000 daltons.

23. The matrix of claim 16, wherein said polysaccharide is selected from the group consisting of heteropolysaccharide, homopolysaccharide and mixture thereof.

24. The matrix of claim 23, wherein said heteropolysaccharide is selected from the group consisting of gellan, welan, gum arabic, karaya gum, okra gum, aloe gum, gum tragacanth, gum ghatti quicessed gum, psyllium, galactans, galactomannans, glucomannans, polyuronic acids, dextran sulfate, heparin, pectin, sodium alginate and starch arabinogalactan.

25. The matrix of claim 24, wherein said galactan is selected from the group consisting of agar, agarose, kappa, carageenan, iota carageenan and lambda carageenan.

26. The matrix of claim 24, wherein said galactomannan is selected from the group consisting of locust bean gum and guar.

27. The matrix of claim 24, wherein said glucan is selected from the group consisting of cellulose and derivatives thereof, starch and derivatives, dextrans, pullulan, beta 1,3-glucans, chitin, xanthan and tamatind.

28. The matrix of claim 24, wherein said glycomannan is konjac.

29. The matrix of claim 24, wherein said polyuronic acid is selected from the group consisting of algin, alginate and pectin.

30. The matrix of claims 24, wherein said homopolysaccharide is cellulose.

31. The matrix of claim 11, further comprising one or more bacteria is selected from the group consisting of *Bifidobacterium adolescentis, Bifidobacterium angulatum, Bifidobacterium animalis, Bifidobacterium asteroides, Bifidobacterium bifidum, Bifidobacterium boum, Bifidobacterium breve, Bifidobacterium catanulatum, Bifidobacterium choerinum, Bifidobacterium coryneforme, Bifidobacterium cuniculi, Bifidobacterium dentium, Bifidobacterium gallicum, Bifidobacterium gallinarum, Bifidobacterium indicum, Bifidobacterium infantis, Bifidobacterium longum, Bifidobacterium longum* DJOIOA, *Bifidobacterium longum* NCC2705, *Bifidobacterium magnum, Bifidobacterium merycicum, Bifidobacterium minimum, Bifidobacterium pseudocatenulatum, Bifidobacterium pseudolongum, Bifidobacterium pseudolongum subsp. globosum, Bifidobacterium pullorum, Bifidobacterium ruminantium, Bifidobacterium saeculare, Bifidobacterium scardovii, Bifidobacterium subtitle, Bifidobacterium suis, Bifidobacterium thermacidophilum, Bifidobacterium thermacidophilum subsp. suis, Bifidobacterium thermophilum, Bifidobacterium urinalis, Lactobacillus acetotolerans, Lactobacillus acidipiscis, Lactobacillus acidophilus, Lactobacillus agilis, Lactobacillus algidus, Lactobacillus alimentarius, Lactobacillus amylolyticus, Lactobacillus amylophilus, Lactobacillus amylovorus, Lactobacillus animals, Lactobacillus arizonenis, Lactobacillus aviarvius, Lactobacillus bifermentans; Lactobacillus brevis, Lactobacillus buchneri, Lactobacillus casei, Lactobacillus cellobiosus, Lactobacillus coleohominis, Lactobacillus collinoides, Lactobacillus coryniformis, Lactobacillus coryniformis subsp. coryniformis, Lactobacillus corniformis subsp. torquens, Lactobacillus crispatus, Lactobacillus curvatus, Lactobacillus cypricasei, Lactobacillus delbrueckii, Lactobacillus delbrueckii subsp. bulgaricus, Lactobacillus delbrueckii subps. delbrueckii, Lactobacillus delbrueckii subsp, lactis, Lactobacillus durianis, Lactobacillus equi, Lactobacillus farciminis, Lactobacillus ferintoshensis, Lactobacillus fermentum, Lactobacillus fornicalis, Lactobacillus fructivorans, Lactobacillus frumenti, Lactobacillus fuchuensis, Lactobacillus gallinarum, Lactobacillus gasseri, Lactobacillus graminis, Lactobacillus hamsteri, Lactobacillus helveticus, Lactobacillus helveticus subsp. jugurti, Lactobacillus heterohiochii, Lactobacillus hilgardii, Lactobacillus homohiochii, Lactobacillus intestinalis, Lactobacillus japonicus, Lactobacillus jensenii, Lactobacillus johnsonii, Lactobacillus kefer, Lactobacillus kefiri, Lactobacillus kefiranofaciens, Lactobacillus kefirgranum, Lactobacillus kimchii, Lactobacillus kunkeei, Lactobacillus leichmannii, Lactobacillus letivazi, Lactobacillus lindneri, Lactobacillus malefermentans, Lactobacillus mali, Lactobacillus maltaromicus, Lactobacillus manihotivorans, Lactobacillus mindensis, Lactobacillus mucosae, Lactobacillus murinus, Lactobacillus nagelii, Lactobacillus oris, Lactobacillus panis, Lactobacillus pantheris, Lactobacillus parabuchneri, Lactobacillus paracasei, Lactobacillus paracasei subsp. paracasei, Lactobacillus paracasei subsp. tolerans, Lactobacillus parakefiri, Lactobacillus paralimentarius, Lactobacillus paraplantarum, Lactobacillus pentosus, Lactobacillus perolens, Lactobacillus plantarum, Lactobacillus pontis, Lactobacillus psittaci, Lactobacillus reuteri, Lactobacillus rhamrrosus, Lactobacillus ruminis, Lactobacillus sakei, Lactobacillus sakei* L45, *Lactobacillus salivarius, Lactobacillus salivarius subsp. salicinius, Lactobacillus salivarius subsp. salivarius, Lactobacillus sanfranciscensis, Lactobacillus sharpeae, Lactobacillus sp.* NGRI 0001, *Lactobacillus suebicus, Lactobacilluss thermotolerans, Lactobacillus vaccinostercus, Lactobacillus vaginalis, Lactobacillus vermiforme, Lactobacillus versmoldensis, Lactobacillus zeae, Lactococcus garvieae, Lactococcus lactis, Lactococcus lactis subsp. cremoris, Lactococcus lactis subsp. hordniae, Lactococcus lactis subsp. lactis, Lactococcus latis subsp. lactis bv. diacetylactis, Lactococcus piscium; Lactococcus plantarum, Lactococcus raffinolactis, Leuconostoc argentinum, Leuconostoc carnosum, Leuconostoc citreum, Leuconostoc fallax, Leuconostoc ficulneum, Leuconostoc fructosum, Leuconostbo gasicomitatum, Leuconostoc gelidum, Leuconostoc inhae, Leuconostoc kimchii, Leuconostoc lactis, Leuconostoc mesenteroides, Leuconstoc mesenteroides subsp. cremoris, Leuconostoc mesenteroides subsp. dextranicum, Leuconostoc mesenteroides subsp. mesenteroides, Leuconostoc mesenteroides subsp. mesenteroides* ATCC 8293, *Leuconostoc pseudomesenteroides, Propionibacterium acidipropionici, Propionibacterium acnes; Propionibacterium australiense, Propionobacterium avidum_{;} Propionibacterium cyclohexanicum, Propionibacterium freudenreichii, Propionibacterium freudenreichii subsp. freudenreichii, Propionibacterium freudenreichii subsp. shermanii, Propionibacterium granulosum, Propionibacterium jensenii, Propionobacterium lumphophilum; Propionibacterium microaerophilum; Propionibacterium propionicum and Propionibacterium thoenii.*

32. A microorganism R2C2 isolated from a consortium obtained from Kefir grain under NML accession number 041202-3.

33. A microorganism K2 isolated from a consortium obtained from Kefir grain under NML accession number 041202-1.

34. A microorganism ES1 isolated from a consortium obtained from Kefir grain under NML accession number 041202-2.

35. A microorganism INIX isolated from ATCC 43761 strain.

36. A composition comprising the matrix of any one of claims 11 to 31 in association with a pharmaceutically acceptable carrier.

37. Use of the matrix of any one of claims 11 to 31, wherein said use is for the manufacture of a product selected from the group of food product, medical product, pharmaceutical product, cosmetic product and nutraceutical.

38. Use of the matrix of any one of claims 11 to 31, wherein said use is for the manufacture of a food product.

39. The use as claimed in claim 38, wherein said matrix is used as an emulsion stabilizer or thickening agent.

40. The use as claimed in claim 37 and 38, wherein said food product is selected from the group consisting of mayonnaise, dressing, margarine, spread, butter, whipped cream and low-fat substitute.

41. The use as claimed in claim 37, wherein said matrix is used as a delivery vehicle.

42. Use of the matrix of any one of claims 11 to 31, wherein said use is for cosmetic product.

43. The use as claimed in claim 42, wherein said cosmetic product is selected from the group consisting of skin lotion, cream, sunscreen, blush, mascara, eyeshadow, shampoo and conditioner.

44. Use of the matrix of any one of claims 11 to 31 for the manufacture of a medicament for increasing immune response in a subject.

45. Use of the matrix of any one of claims 11 to 31 for the manufacture of a medicament for reducing triclyceride level in a subject.

46. Use of the matrix of anyone of claims 11 to 31 for the manufacture of a medicament for reducing TNF-α level in a subject.

47. Use of the matrix of any one of claims 11 to 31 for the manufacture of a medicament for increasing gluthatione level in a subject.

## Patentansprüche

1. Verfahren zur Herstellung einer formbaren Proteinmatrix, wobei das Verfahren die folgenden Schritte umfasst:
a) Fermentieren einer Molkeproteinlösung mit Bakterien in einem Medium;
b) Agglomerieren von Proteinen aus der Proteinlösung von Schritt a); und
c) Isolieren der agglomerierten Proteine aus dem Überstand;
**dadurch gekennzeichnet, dass** die Bakterien mindestens ein Bakterium, das aus R2C2, INIX, ES1 und K2 ausgewählt ist, umfassen

2. Verfahren nach Anspruch 1, wobei der Fermentationsschritt durch gleichzeitige oder aufeinanderfolgende Cokultivierung von mindestens zwei Mikroorganismen gefördert wird.

3. Verfahren nach Anspruch 1, wobei das Verfahren ferner einen Schritt zwischen den Schritten a) und b) für die Zugabe eines Polysaccharids umfasst.

4. Verfahren nach Anspruch 1, wobei das Verfahren ferner einen Schritt zwischen den Schritten b) und c)für die Zugabe eines Polysaccharids umfasst.

5. Verfahren nach einem der Ansprüche 1 bis 4, wobei die Agglomeration von fermentierten Proteinen durch mindestens eine Methode bewirkt wird, die aus der Gruppe bestehend aus Salzzugabe, pH-Modulation, Wärmebehandlung, Zugabe von proteolytischen Enzymen und Flockungsmitte-zugabe ausgewählt ist.

6. Verfahren nach Anspruch 2, ferner umfassend einen Schritt der Pasteurisierung der Proteinlösung vor Schritt a).

7. Verfahren nach Anspruch 6, wobei dem Schritt der Pasteurisierung ein Schritt der Sterilisation folgt.

8. verfahren nach Anspruch 5, wobei das Flockungsmittel ein bakterielles Flockungsmittel ist.

9. Verfahren nach Anspruch 8, wobei das bakterielle Flockungsmittel *L. kefirgranum* ist.

10. Verfahren nach einem der Ansprüche 1 bis 9, wobei Trennung von agglomerierten Proteinen von dem Überstand durch eine Methode bewirkt wird, die aus der Gruppe von Zentrifugieren und Filtration ausgewählt ist.

11. Verformbare Proteinmatrix, die durch das Verfahren von Anspruch 1 erhältlich ist.

12. Matrix nach Anspruch 11, ferner umfassend ein Fermentationsnebenprodukt der Fermentation der Lösung, die das Protein enthält, durch die Bakterien.

13. Matrix nach Anspruch 11, ferner umfassend ein Peptid.

14. Matrix nach Anspruch 13, wobei das Peptid mindestens zwei Aminosaurereste umfasst.

15. Matrix nach Anspruch 13, wobei das Peptid mehr als hundert Aminosäurereste umfasst.

16. Matrix nach Anspruch 12, wobei das Fermentationsnebenprodukt ein Polysaccharid ist.

17. Matrix nach Anspruch 16, wobei das Polysaccharid aus der Gruppe von Exopolysaccharid und anionischem Polysaccharid ausgewählt ist.

18. Matrix nach Anspruch 16, wobei das Polysaccharid mindestens vier Saccharideinheiten enthält.

19. Matrix nach Anspruch 18, wobei die Saccharideinheiten aus der Gruppe bestehend aus D- und L-Formen von Glucose, Fructose, Xylose, Arabinose, Fucose, Galactose, Brenztraubensäure, Bernsteinsäure, Essigsäure, 3,6-Anhydrogalactosesulfat, Galactose-4-sulfat, Galactose-2-sulfat, Galactose-2,6-disulfat, Mannose, Glucuronsäure, Mannuronsäure, Guluronsäure, Galacturonsäure und Rhamnose ausgewählt sind.

20. Matrix nach Anspruch 16, wobei das Polysaccharid ein Molekulargewicht im Bereich von etwa 500 bis etwa 15.000.000 Dalton aufweist.

21. Matrix nach Anspruch 16, wobei das Molekulargewicht im Bereich von etwa 5000 bis 6.000.000 Dalton liegt.

22. Matrix nach Anspruch 16, wobei das Molekulargewicht im Bereich von etwa 25.000 bis 1.000.000 Dalton liegt.

23. Matrix nach Anspruch 16, wobei das Polysaccharid aus der Gruppe bestehend aus Heteropolysaccharid, Homopolysaccharid und Mischungen davon ausgewählt ist.

24. Matrix nach Anspruch 23, wobei das Heteropolysaccharid aus der Gruppe bestehend aus Gellan, Welan, Gummi arabicum, Karaya-Gummi, Okra-Gummi, Aloe-Gummi, Tragant-Gummi, Ghatti-Gummi, Quittensamen-Gummi, Psyllium, Galactane, Galactomannane, Glucomannane, Polyuronsäure, Dextransulfat, Heparin, Pektin, Natriumalginat und Stärke-Arabinogalactan ausgewählt ist.

25. Matrix nach Anspruch 24, wobei das Galactan aus der Gruppe bestehend aus Agar, Agarose, Kappa, Carrageen, Jota-Carrageen und Lambda-Carrageen ausgewählt ist.

26. Matrix nach Anspruch 24, wobei das Galactomannan aus der Gruppe bestehend aus Johannisbrotkernmehl und Guar ausgewählt ist.

27. Matrix nach Anspruch 24, wobei das Glucan aus der Gruppe bestehend aus Cellulose und deren Derivaten, Stärke und Derivaten, Dextranen, Pullulan, Beta-1,3-Glucanen, Chitin, Xanthan und Tamatind ausgewählt ist.

28. Matrix nach Anspruch 24, wobei das Glycomannan Konjak ist.

29. Matrix nach Anspruch 24, wobei die Polyuronsäure aus der Gruppe bestehend aus Algin, Alginat und Pektin ausgewählt ist.

30. Matrix nach Anspruch 24, wobei das Homopolysaccharid Cellulose ist.

31. Matrix nach Anspruch 11, ferner umfassend eines oder mehrere Bakterien, die aus der Gruppe bestehend aus *Bifidobacterium, adolescentis, Bidobacterium angulatum Bifidobacterium animalis, Bifidobacterium asteroides, Bfdobacterium bifidum, Bifidobacterium boum, Bifidobacterium breve, Bifidobacterium catanulatum, Bifidobacterium choerinum, Bifidobacterium coryneformer, Bifidobacterium cuniculi, Bifidobacterium dentium, Bifobacterium gallicum, Bifidobacterium gallinarum, Bifidobacterium indicum, Bifidobacterium infantis, Bifidobacterium longum, Bifidobacterium longum* DJO10A, *Bifidobacterium longum* NCC2705, *Bifidobacterium magnum, Bifidobacterium merycicum, Bifidobacterium minimum, Bifidobacterium pseudocatenulatum, Bifidobacterium pseudolongum, Bifidobacterium pseudolongum subsp. globosum, Bifidobacterium pullorum, Bifidobacterium ruminantium, Bifidobacterium saeculare, Bifidobacterium scardovii, Bifidobacterium subtile, Bifidobacterium suis, Bifidobacterium thermacidophilum, Bifidobacterium thermacidophilum subsp. suis, Bifidobacterium thermophilum, Bifidobacterium urinalis, Lactobacillus acetotolerans, Lactobacillus acidipiscis, Lactobacillus acidophilus, Lactobacillus agilis, Lactobacillus algidus, Lactobacillus alimentarius, Lactobacillus amylclyticus, Lactobacillus amylophilus, Lactobacillus amylovorus, Lactobacillus animalis, Lactobacillus arizonenis, Lactobacillus aviarius, Lactobacillus bifermentans, Lactobacillus brevis, Lactobacillus buchneri, Lactobacillus casei, Lactobacillus cellobiosus, Lactobacillus coleohominis, Lactobacillus collinoides, Lactobacillus coryniforrnis, Lactobacillus coryniformis subsp. coryniformis, Lactobacillus corniformis subsp. torquens, Lactobacillus crispatus, Lactobacillus curvatus, Lactobacillus cypricasei, Lactobacillus delbrueckii, Lactobacillus delbrueckii, subsp. bulgaricus, Lactobacillus delbrueckü subps. delbrueckii, Lactobacillus delbrueckii subsp. lactis, Lactobacillus durianis, Lactobacillus equi, Lactobacillus farciminis, Lactobacillus ferintoshensis, Lactobacillus, fermentum, Lactobacillus, fornicalis, Lactobacillus fructivorans, Lactobacillus frumenti, Lactobacillus fuchuensis, Lactobacillus gallinarum, Lactobacillus gasseri, Lactobacillus graminis, Lactobacillus hamsteri, Lactobacillus helveticus, Lactobacillus helveticus subsp, jugurti, Lactobacillus heterohicchii, Lactobacillus hilgardii, Lactobacillus honzohiochii, Lactobacillus intestinalis, Lactobacillus japonicus, Lactobacillus jensenii, Lactobacillus johnsonii, Lactobacillus kefer, Lactobacillus kefiri, Lactobacillus kefiranofaciens, Lactobacillus kefirgranum, Lactobacillus kimchii, Lactobacillus kunkeei, Lactobacillus leichmannii, Lactobacillus letivazi, Lactobacillus lindneri, Lactobacillus malefermentans, Lactobacillus mali, Lactobacillus maltaromicus, Lactobacillus manihotivorans, Lactobacillus mindensis, Lactobacillus mucosae, Lactobacillus murinus, Lactobacillus nagelii, Lactobacillus oris, Lactobacillus panis, Lactobacillus pantheris, Lactobacillus parabuchneri, Lactobacillus paracasei, Lactobacillus paracasei subsp. paracasei, Lactobacillus paracasei subsp, tolerans, Lactobacillus parakefiri, Lactobacillus paralimentarius, Lactobacillus paraplantarum, Lactobacillus pentosus, Lactobacillus perolens, Lactobacillus plantarum, Lactobacillus pontis, Lactobacillus psittaci, Lactobacillus reuteri, Lactobacillus rhamnosus, Lactobacillus ruminis, Lactobacillus sakei, Lactobacillus sakei* L45, *Lactobacillus salivarius, Lactobacillus salivarius subsp. salicinius, Lactobacillus salivarius subsp. salivarius, Lactobacillus sanfranciscensis, Lactobacillus sharpeae, Lactobacillus sp.* NGRI 0001, *Lactobacillus suebicus, Lactobacillus thermotolerans, Lactobacillus vaccinostercus, Lactobacillus vaginalis, Lactobacillus vermiforme, Lactobacillus versmoldensis, Lactobacillus zeae, Lactococcus garvieae, Lactococcus lactis, Lactococcus lactis subsp. cremoris, Lactococcus lactis subsp. hordniae, Lactococcus lactis subsp. lactis, Lactococcus latis subsp. lactis bv. diacetylactis, Lactococcus piscium, Lactococcus plantarum, Lactococcus raffinolactis, Leuconostoc argentinum, Leuconostoc carnosum, Leuconostoc citreum, Leuconostoc fallax, Leuconostoc ficulneum, Leuconostoc fructosum, Leuconostoc gasicomitatum, Leuconostoc gelidum, Leuconostoc inhae, Leuconostoc kimchii, Leuconostoc lactis, Lecconostoc mesenteroides, Leuconostoc mesenteroides subsp. cremoris, Leuconostoc mesenteroides subsp. dextranicum, Leuconostoc mesenteroides subsp. mesenteroides, Leuconostoc mesenteroides subsp. mesenteroides* ATCC 8293, *Leuconostoc pseudomosanteroides, Propionibacterium acidipropionici, Propionibacterium acnes, Propionibacterium australiense, Propionibacterium avidum, Propionibacterium cyclohexanicum, Propionibacterium freudenreichii, Propionibacterium freudenreichli subsp. freudenreichii, Propionibacterium freudenreichii subsp, shermanii, Propionibacterium granulosum, Propionibacterium jensenii, Propionibacterium lumphophilum, Propionibacterium microaerophilum, Propionibacterium propionicum und Propionibacterium thoenii.*

32. Mikroorganismus R2C2, das von einem aus Kefirkorn erhaltenen Konsortium, NML Akzessionsnummer 041202-3, isoliert wird.

33. Mikroorganismus K2, das von einem aus Kefirkorn erhaltenen Konsortium, NML Akzessionsnummer 041202-1, isoliert wird.

34. Mikroorganismus ES1, das von einem aus Kefirkorn erhaltenen Konsortium, NML Akzessionsnummer 041202-2, isoliert wird.

35. Mikroorganismus INIX, isoliert von Stamm ATCC 43761.

36. Zusammensetzung, welche die Matrix nach einem der Ansprüche 11 bis 31 in Verbindung mit einem pharmazeutisch annehmbaren Träger umfasst.

37. Verwendung der Matrix nach einem der Ansprüche 11 bis 31, wobei die Verwendung zur Herstellung eines Produkts ist, das aus der Gruppe von Nahrungsmittel, medizinisches Produkt, pharmazeutisches, kosmetisches Produkte und Nutrazeutikum ausgewählt ist.

38. Verwendung der Matrix nach einem der Ansprüche 11 bis 31, wobei die Verwendung zur Herstellung eines Nahrungsmittel ist.

39. Verwendung nach Anspruch 38, wobei die Matrix als Emulsionsstabilisator oder Verdickungsmittel verwendet wird.

40. Verwendung nach Anspruch 37 und 38, wobei das Nahrungsmittel aus der Gruppe bestehend aus Mayonnaise, Dressing, Margarine, Aufstrich, Butter, Sahne und fettarmem Ersatzstoff ausgewählt ist.

41. Verwendung nach Anspruch 37, wobei die Matrix als Abgabevehikel verwendet wird.

42. Verwendung der Matrix nach einem der Ansprüche 11 bis 31, wobei die Verwendung für kosmetisches Produkt ist.

43. Verwendung nach Anspruch 42, wobei das kosmetische Mittel aus der Gruppe bestehend aus Haut-Lotion, Creme, Sonnencreme, Rouge, Mascara, Lidschatten, Shampoo und Haarspülung ausgewählt ist.

44. Verwendung der Matrix nach einem der Ansprüche 11 bis 31 zur Herstellung eines Medikaments zur Steigerung der Immunantwort bei einem Probanden.

45. Verwendung der Matrix nach einem der Ansprüche 11 bis 31 zur Herstellung eines Medikaments zur Serkung des Triglyceridspiegels bei einem Probanden.

46. Verwendung der Matrix nach einem der Ansprüche 11 bis 31 zur Herstellung eines Medikaments zur Senkung des TNF-α-Spiegels bei einem Probanden.

47. Verwendung der Matrix nach einem der Ansprüche 11 bis 31 zur Herstellung eines Medikaments zur Erhöhung des Glutathionspiegels bei einem Probanden.

## Revendications

1. Procédé de préparation d'une matrice protéique malléable, ledit procédé comprenant les étapes de :
a) fermentation d'une solution protéique de lactosérum avec des bactéries dans un milieu.
b) agglomération des protéines de la solution protéique de l'étape a); et
c) isolation des protéines agglomérées du surnageant;
**caractérisé en ce que** lesdites bactéries comprennent au moins une bactérie choisie dans le groupe constitué de R2C2, INIX, ES1 et K2.

2. Le procédé tel que défini à la revendication 1, **caractérisé en ce que** l'étape de fermentation est réalisée en présence d'une co-culture simultanée ou successive d'au moins deux microorganismes.

3. Le procédé tel que défini à la revendication 1, **caractérisé en ce que** ledit procédé comprend en outre une étape entre les étapes a) et b) pour l'addition d'un polysaccaride.

4. Le procédé tel que défini à la revendication 1, **caractérisé en ce que** ledit procédé comprend en outre une étape entre les étapes b) et c) pour l'addition d'un polysaccaride.

5. Le procédé tel que défini à l'une quelconque des revendication 1 à 4, **caractérisé en ce que** l'agglomération des protéines fermentées est effectuée par au moins une méthode choisie dans le groupe constitué de l'addition d'un sel, de la modulation du pH, d'un traitement thermique, d'un addition dans une protéolitique et d'un addition de floculant.

6. Le procédé tel que défini à la revendication 2, comprenant en outre une étape de pasteurisation de ladite solution protéique avant l'étape a).

7. Le procédé tel que défini à la revendication 6, caractérisé en ce l'étape de pasteurisation est suivie d'une étape de stérilisation.

8. Le procédé tel que défini à la revendication 5, caractérisé en ce ledit floculant est une bactérie floculante.

9. Le procédé tel que défini à la revendication 8, **caractérisé en ce que** la bactérie floculante est *L. Kefirgranum.*

10. Le procédé tel que défini à l'une quelconque des revendications 1 à 9, **caractérisé en ce que** la séparation des protéines agglomérées du surnageant est effectuée par une méthode choisie parmi une centrifugation et une filtration.

11. Une matrice protéique malléable susceptible d'être obtenue par la procédé tel que défini à la revendication 1.

12. La matrice telle que définie à la revendication 11, comprenant en outre un sous-produit de fermentation de la fermentation de la solution protéique par ladite bactérie,

13. La matrice telle que définie à la revendication 11, comprenant en outre un peptide.

14. La matrice telle que définie à la revendication 13, **caractérisée en ce que** ledit peptide comprend au moins deux résidus d'acide aminé.

15. La matrice telle que définie la la revendication 13, **caractérisée en ce que** ledit peptide comprend plus de cent résidus d'acide aminé.

16. La matrice telle que définie à la revendication 12, **caractérisée en ce que** ledit sous-produit de fermentation est un polysaccharide.

17. La matrice telle que définie à la revendication 16, **caractérisée en ce que** ledit polysaccharide est sélectionné parmi un exopolysaccharide et un polysaccharide anionique.

18. La matrice telle que définie à la revendication 16, **caractérisée en ce que** ledit polysaccharide contient au moins quatre groupement saccharidique.

19. La matrice telle que définie à la revendication 18, **caractérisée en ce que** lesdits groupements saccharidiques sont sélectionnés parmi le groupe de forme D et L du glucose, fructose, xylose, arabinose, fucose, galactose, acide pyruvique, acide succinique, sulfate de 3,6-anhydrogalacose, galactose-4-sulfate, galactose-2-sulfate, galactose-2,6-disulfate, mannose, acide glucuronique, acide mannuronique, acide guluronique, acide galactouronique et rhamnose.

20. La matrice telle que définie à la revendication 16, **caractérisée en ce que** ledit polysaccharide a un poids moléculaire variant d'environ 500 à environs 15,000,000 daltons.

21. La matrice telle que définie à la revendication 16, **caractérisée en ce que** ledit poids moléculaire varie d'environ 5,000 à 6,000,000 daltons.

22. La matrice telle que définie à la revendication 16, **caractérisée en ce que** ledit poids moléculaire varie d'environ 25,000 à 1,000,000 daltons.

23. La matrice telle que définie à la revendication 16, **caractérisée en ce que** ledit polysaccharide est choisi parmi un hétéropolysaccharide, un homopolysaccharide ou un mélange de ces derniers.

24. La matrice telle que définie à la revendication 23, **caractérisée en ce que** ledit hétéropolysaccharide est sélectionné parmi le groupe constitué de gellan, welan, gomme arabique, gomme karaya, gomme okra, gomme aloe, gomme tragacanth, gomme ghatti, gomme quicessed, psyllium, galactanes, galactomannans, glucomannans, acides polyuroniques, sulfate dextran, héparine, peptine, alginate de sodium et arabinogalactan d'amidon

25. La matrice telle que définie à la revendication 24, **caractérisée en ce que** lesdits galactomannans sont choisis parmi le groupe constitué de agar, agarose, kappa carageenan, iota carageenan et lambda carageenan.

26. La matrice telle que définie à la revendication 24, **caractérisée en ce que** lesdits galactomannans sont sélectionnés parmi le groupe constitué par la gomme de caroube et guar.

27. La matrice telle que définie à la revendication 24, **caractérisée en ce que** ledit glucane est sélectionne parmi le groupe constitué de cellulose et de dérivés de cette dernière, d'amidon et dérivés d'amidon, dextranes, pullalan, beta 2,3-glucans, chitin, xanthan et tamatind.

28. La matrice telle que définie à la revendication 24, **caractérisée en ce que** ledit glucomannan est le konjac.

29. La matrice telle que définie a la revendication 24, **caractérisée en ce que** ledit acide polyuronique est choisi parmi le groupe constitué d'algine, d'alginate et de pectine.

30. La matrice telle que définie à la revendication 24, **caractérisée en ce que** ledit homopolysaccharide est la cellulose.

31. La matrice telle que définie à la revendication 11, comprenant en outre une ou plusieurs bactéries choisies dans le groupe constitué de *Bifidobectarium adalescentis,* Bifidobacterium *angulatum, Bifidobacterium animalis, Bifidobacterinum asteroides*, *Bifidobacterium bifidum, Bifidobacterium boum, Bifidobecterium breve, Bifidobacterium catenulatum, Bifidobacterium choerinum, Bifidobacterium coryneforme, Bifidobacterium cuniculi, Bifidobacterium dentium, Bifidobacterium gallicum Bifidobacterium gallinarum, Bifidobecterium indicum, Bifidobacterium infantis, Bifidobecterium longum, Bifidobacterium longum* DJO10A, *Bifidobacterium longum* NCC2705, *Bifidobecterium magnum Bifidobecterium merycicum, Bifidobecterium minimum, Bifidobacterium pseudocatenulatum, Bifidobecterium pseudolongum, Bifidobecterium pseudolongum subsp globosum, Bifidobacterium pullorum, Bifddobacterium ruminantium, Bifidobacterium saeculare, Bifidobacterium scardovii, Bifidobacterium subtile, Bifidobacterium suis, Bifidobacterium thermacidophilum Bifidobacterium thermacidophilum subsp. suis, Bifidobacterium thermophilum, Bifidobacterium urinalis, Lactobacillus acetotolerans, Lactobacillus acidipiscis, Lactobacillus acidophilus, Lactobacillus agilis, Lactobacillus algidus, Lactobacillus alimentarius, Lactobacillus amylolyticus, Lactobacillus amylophilus, Lactobacillus amylovorus, Lactobacillus animalis, Lactobacillus arizonensis, Lactobacillus aviarius, Lactobacillus bifermentans, Lactobacillus brevis, Lactobacillus buchneri, Lactobacillus casei, Lactobacillus cellobiosus, Lactobacillus coleohominis, Lactobacillus collinoides, Lactobacillus coryniformis, Lactobacillus coryniformis subsp. coryniformis, Lactobacillus coryniformis subsp. torquens, Lactobacillus crispatus, Lactobacillus curvatus, Lactobacillus cypricasei, Lactobacillus dalbrueckii, Lactobacillus delbrueckii subsp. bulgaricus, Lactobacillus delbrueckii subsp. delbrueckii, Lactobacillus delbrueckii subsp. lactis, Lactobacillus durianis, Lactobacillus equi, Lactobacillus farciminis, Lactobacillus ferintoshensis, Lactobacillus fermentum, Lactobacillus fornicalis, Lactobacillus fructivorans, Lactobacillus frumenti, Lactobacillus fuchuensis, Lactobacillus gallinarum, Lactobacillus gasseri, Lactobacillus graminis Lactobacillus hamsteri, Lectobacillus helveticus, Lactobacillus helveticus subsp. jugurti, Lactobacillus heterohiochii, Lactobacillus hilgardii, Lactobacillus homohiochii, Lactobacillus intestinalis, Lactobacillus japonicus, Lactobacillus jensenii, Lactobacillus johnsonii, Lactobacillus kefir, Lactobacillus kefiri, Lactobacillus kefiranofaciens, Lactobacillus kefirgranum, Lactobacillus kimchii, Lactobacillus kunkeei, Lactobacillus leichmannii, Lactobacillus letivazi, Lactobacillus lindneri, Lactobacillus malefermentans, Lactobacillus mali, Lactobacillus maltaromicus, Lactobacillus manihotivorans, Lactobacillus mindensis, Lectobacillus mucosae, Lactobacillus murinus, Lactobacillus nagelii, Lactobacillus oris, Lactobacillus panis, Lactobacillus pantheris, Lactobacillus parabuchneri, Lactobacillus paracasei, Lactobacillus paracasei subsp. paracasei, Lactobacillus paracasei subsp tolerans, Lactobacillus parakefiri, Lactobacillus paralimentarius Lactobacillus paraplanterum, Lactobacillus pentosus, Lactobacillus perolens, Lactobacillus plantarum, Lactobacillus pontis Lactobacillus psittaci, Lactobacillus reuteri, Lactobacillus rhamnosus, Lactobacillus ruminis, Lactobacillus sakei, Lactobaclllus sakei* L45 *Lactobacillus salivarius, Lactobacillus salivarius subsp. salicinius, Lactobacillus salivarius subsp. salivarius, Lactobacillus sanfranciscensis, Lactobacillus sharpeae, Lactobacillus sp,* NGRI 0001 *Lactobacillus suebicus, Lactobacillus thermotolerans, Lactobacillus vaccinostercus, Lactobacillus vaginalis, Lactobacillus vermiforme, Lactobacillus versmoldensis, Lactobacillus zeae, Lactococcus garvieae, Lactococcus lactis, Lactococcus lactis subsp. cremoris, Lactococcus lactis subsp. hordniae Lactococcus lactis subsp. lactis, Lactococcus lactis subsp lactis bv. diacetylactis, Lactococcus piscium, Lactococcus plantarum, Lactococcus raffinolactis, Leuconostoc argentinum, Leuconostoc* carnosum, *Leuconostoc citreum, Leuconostoc fallax, Leuconostoc ficulneum, Leuconostoc fructosum, Leuconostoc gasicomitatum, Leuconostoc galidum, Leuconostoc inhae, Leuconostoc kimchii, Leuconostoc lactis, Leuconostoc mesenteroides, Leuconostoc mesenteroides subsp. cremoris, Leuconostoc mesenteroides subsp. dextranicum, Leuconostoc mesenteroides subsp. mesenteroides, Leuconostoc mesenteroides subsp. mesenteroides* ATCC 8293, *Leuconostoc pseudomasenteroides, Propionibacterium acidipropionici, Propionibacterium acnes, Propionibacterium australiense, Propionibacterium avidum, Propionibecterium cyclohexanicum, Propionibacterium freudenreichii, Propionibacterium freudenreichii subsp. freudenreichii, Propionibacterium freudenreichii subsp. shermanii*, *Propionibacterium granulosum, Propionibacterium jensenii, Propionibacterium iymphophilum, Propionibacterium microaerophilum, Propionibacterium propionicum,* et *Propionibacterium thoenii.*

32. Microorganisme R2C2 isolé à partir d'un consortium obtenu de la graine de Kéfir, ayant un numéro d'accession NML 041202-3.

33. Microorganisme K2 isolé à partir d'un consortium obtenu de la graine de Kéfir, ayant un numéro d'accession NML 041202-1.

34. Microorganisme ES1 Isolé à partir d'un consortium obtenu de la graine de Kéfir, ayant un numéro d'accession NML 041202-2.

35. Microorganisme INIX isolé de la soucheATCC 43761.

36. Une composition comprenant la matrice telle que définie dans l'une quelconque des revendications 11 à 31 en association avec un excipient pharmaceutiquement acceptable.

37. Usage de la matrice telle que définie à l'une quelconque des revendications 11 à 31. **caractérisé en ce que** ledit usage est pour la production d'un produit choisi dans le groupe constitué de produit alimentaire, produit médical, produit pharmaceutique, produit cosmétique et produit nutraceutique.

38. Usage de la matrice telle que définie à l'une quelconque des revendications 11 à 31, **caractérisé en ce que** ledit usage est pour la fabrication d'un produit alimentaire.

39. Usage tel que défini à la revendication 38, **caractérisé en ce que** la matrice est utilisée comme agent stabilisant une émulsion ou comme agent épaississant.

40. Usage tel que défini à la revendication 37 ou 38, **caractérisé en ce que** ledit produit alimentaire est sélectionné parmi le groupe constitué de mayonnaise, assaisonnement, margarine, tartinade, beurre, crème fouettée et substitut faible en gras.

41. Usage tel que défini à la revendication 37, **caractérisé en ce que** la matrice est utilisée comme un agent de livraison.

42. Usage de la matrice telle que définie à l'une quelconque des revendications 11 à 31, **caractérisé en ce que** ledit usage est pour un produit cosmétique.

43. Usage tel que défini à la revendication 42, **caractérisé en ce que** ledit produit cosmétique est choisi parmi le groupe constitué de lotion pour la peau, crème, crème solaire, fard à joues, mascara, fard à paupières, shampoo et revitalisant.

44. Usage de la matrice telle que définie à l'une quelconque des revendications 11 à 31 pour la fabrication d'un médicament pour augmenter une réponse immunitaire chez un patient.

45. Usage de la matrice telle que définie à l'une quelconque des revendications 11 à 31 pour la fabrication d'un médicament pour réduire le taux de triglycéride chez un sujet.

46. Usage de la matrice telle que définie à l'une quelconque des revendications 11 à 31 pour la fabrication d'un médicament pour réduire le taux de TNF-α chez un sujet.

47. Usage de la matrice telle que définie à l'une quelconque des revendications 11 à 31 pour la fabrication d'un médicament pour augmenter le taux de glutathione chez un patient.
